# EUROPEAN PATENT SPECIFICATION

(11) **EP 2 086 539 B1**
(45) Date of publication and mention of the grant of the patent: **25.12.2013**
(21) Application number: 07848384.9
(22) Date of filing: 15.11.2007
(51) Int. Cl.: A61K 31/437, C07D 471/04, A61P 5/50, A61P 25/28

(54) **SELECTIVE GLYCOSIDASE INHIBITORS**
SELEKTIVE GLYKOSIDASE-HEMMER
INHIBITEURS DE GLYCOSIDASES SÉLECTIFS

(30) Priority: 15.11.2006 GB 0622702
(43) Date of publication of application: 12.08.2009
(73) Proprietor: The University Court of the University of Dundee, Dundee DD1 4AS (GB)
(72) Inventor: VAN AALTEN, Daniel Marinus, Dundee DD1 5EH (GB); BORODKIN, Vladimir Sergeevich, Dundee DD1 5EH (GB); DORFMULLER, Helge, Dundee DD1 5EH (GB)
(74) Representative: Horner, Martin Grenville
(86) International application number: PCT/GB2007/004384
(87) International publication number: WO 2008/059267

(56) References cited:
- WO-A-2005/046611
- WO-A-2006/092049
- SHANMUGASUNDARAM BHAGAVATHY ET AL: "Inhibition of O-GlcNAcase by a gluco-configured nagstatin and a PUGNAc-imidazole hybrid inhibitor." CHEMICAL COMMUNICATIONS (CAMBRIDGE, ENGLAND) 13 NOV 2006, no. 42, 13 November 2006 (2006-11-13), pages 4372-4374, XP002473626 ISSN: 1359-7345 cited in the application
- DORFMUELLER HELGE C ET AL: "GlcNAcstatin: A picomolar, selective O-GlcNAcase inhibitor that modulates intracellular O-GlcNAcylation levels" JOURNAL OF THE AMERICAN CHEMICAL SOCIETY, vol. 128, no. 51, December 2006 (2006-12), pages 16484-16485, XP002473627 ISSN: 0002-7863
- TERINEK ET AL.: "Synthesis of N-Acetylglucosamine-derived Nagstatin analogues and thier evaluation as glycosidase inhibitors", HELVETICA CHIMICA ACTA, vol. 88, 2005, pages 10-22,

## Description

### Field of the invention

The present invention relates to compounds for the selective inhibition of glycosidase enzymes, methods of manufacture of the compounds, and the use of these compounds in the treatment of diseases or disorders responsive to inhibition of glycosidases.

The invention is limited to the subject-matter defined in the claims; the following description is subject to this limitation.

### Background to the invention

Many proteins in the eukaryotic cell are modified by *O-*linked N-acetylglucosamine (*O*-GlcNAc) on serines and threonines¹. *O*-GlcNAcylation has been shown to be important for regulation of the cell cycle, DNA transcription and translation, insulin sensitivity and protein degradation^{2,3}. Misregulation of *O*-GlcNAcylation is associated with diabetes and Alzheimer's disease^{2,4,5}. Two enzymes are involved in the dynamic cycling of this posttranslational modification, the *O*-GlcNAc transferase (OGT, classified as CAZY⁶ family GT41) and *O*-GlcNAcase (OGA, GH84). PUGNAc (*O-*(2-acetamido-2-deoxy-D-glucopyrano-sylidene)amino-N-phenylcarbamate), a nanomolar inhibitor of OGA, has been extensively used to induce and study the effects of raised O-GlcNAc levels in the cell^{7,8}. However, PUGNAc is also a potent inhibitor of the human lysosomal hexosaminidases HexA and HexB, inactivation of which has been associated with the Tay-Sachs and Sandhoff lysosomal storage disorders. Therefore PUGNAc lacks selectivity and so is not a suitable candidate for therapeutic uses. While more selective derivatives of PUGNAc and other OGA inhibitors have recently been reported^{9,10,11}, these are also associated with weaker (micromolar) inhibition of OGA. Very recently, a new glucoimidazole compound (A, below), related to the naturally occurring gluco-nagstatin OGA inhibitor has been reported^{11a}. However this also only acts at the micromolar level and its effect on hexosaminidases is unreported.

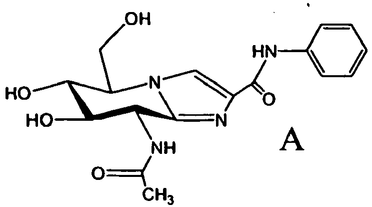

In WO2006/092049 (Simon Fraser University) alternative glycosidase inhibiting compounds are proposed, together with suggestions for their use in the study of diseases and disorders relating to deficiency or overexpresssion of *O-*GlcNAcase, accumulation or deficiency of *O*-GlcNAc, and treatment of such disorders and diseases. The use of the glycosidase inhibitors disclosed is suggested for the treatment of Alzheimers Disease, diabetes and cancer.

For example, it is proposed in WO2006/092049 that compounds that selectively inhibit *O*-GlcNAcase without substantial inhibition of the hexosaminidases A and B can be used to block the hyperphosphorylation of the "tau" protein that is associated with the formation of the neurofibrillary tangles (NFTs) found in the brains of Alzheimers Disease patients. As the NFT level correlates to the severity of the dementia, such selective inhibitors may provide a treatment for the disease.

It is an objective of the present invention to provide further selective inhibitors of glycosidases.

### Description of the invention

According to a first aspect the present invention provides the use of a compound of general formula I: 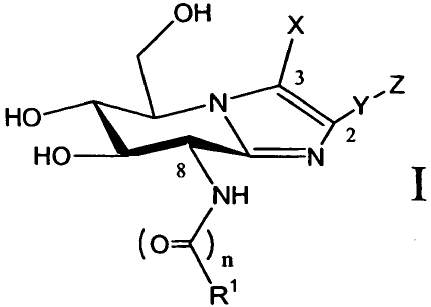 wherein n is 0 or 1;
X is hydrogen, halogen, -OH, -SH, or -NH₂; and
R¹ is a C₁ - C₆ saturated or unsaturated aliphatic chain which may be branched, a five membered, saturated or unsaturated ring, comprising from one to four nitrogen atoms or a substituted derivative thereof, 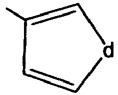 where d is CH₂, O or S; or, when n is 1, 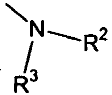 where R², R³ are independently hydrogen or C₁ - C₅ alkyl or are fused to form a saturated ring with the nitrogen which has from two to five carbon atoms;
-Y- is a linking group selected from the group consisting of -CH₂CH₂-, -CH=CH-, -C≡C-, -CH₂O-, -CH₂S- and CH₂NH-; and
-Z is a hydrophobic group or an oligopeptide chain;
in the preparation of a medicament for the treatment or prophylaxis of a condition mediated by the inhibition of O-GlcNAcase enzymes.

Preferably the linking group -Y- is -CH₂CH₂-.

The group Z may be a hydrophobic group. In crystal structure studies of a compound of the invention (compound II) below, in place on the active site of the OGA enzyme, it has been shown that the group Z extends away from the active site (a pocket in the enzyme) interacting with a solvent exposed Trp residue which has a hydrophobic character. Preferably the hydrophobic group -Z is aryl, substituted aryl, heteroaryl, substituted heteroaryl, C₁-C₆ saturated or unsaturated alkyl which may be branched, C₅ - C₆ saturated or unsaturated cycloalkyl or -COOR⁴ where R⁴ is C₁ - C₆ saturated or unsaturated alkyl which may be branched, aryl, or heteroaryl. The term aryl refers to C₆-C₁₄ aromatic radicals such as phenyl,naphthyl, tetrahydronaphthyl, indanyl and biphenyl. Heteroaryl substituents have C₆-C₁₄ aromatic radicals comprising at least one atom that is not carbon. Where the aryl or heteroaryl groups are substituted the substituents may be one or more of alkyl, halogen, hydroxyl, carboxyl, carboxylate ester, cyano, amino, nitro, oxo, or thio. Preferably where -Z is aryl it is phenyl or substituted phenyl.

Alternatively Z is an oligopeptide chain. Preferably the oligopeptide chain comprises 4 to 15 amino acid residues. Preferably the amino acid sequence of the chain corresponds to fragments of the naturally occurring peptides featuring O-GlcNAc modification.

Where R¹ is a five membered ring containing one or more nitrogens preferred structures include 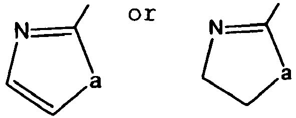 where a is O,S or NH.

Advantageously, when n is 1 and R¹ is aliphatic and unsaturated it has the form, 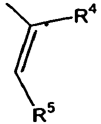 wherein R4 and R5 are, independently, hydrogen or methyl.

The compounds of general formula I are structurally related to the naturally occurring hexosaminidase inhibitor nagstatin . Nagstatin and related glycoimidazole compounds with variable configuration of the sugar ring have been synthesised by Tatsuta and shown to have up to nanomolar inhibition against various glycosidase inhibitors ^{14,15,16,17}. Other nagstatin analogues have been prepared and studied by the Vasella group^{13,18}.

The compounds of general formula I can show inhibition, at down to picomolar levels, for the inhibition of O-GlcNAcase with great selectivity over inhibition of the hexosaminidases (HexA and HexB). They can therefore be described as 'O-GlcNAcstatins'.

In a preferred example of the compound of formula I, X is hydrogen, -Y-Z is phenethyl, n is 1 and R¹ is 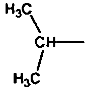 as in formula II below.

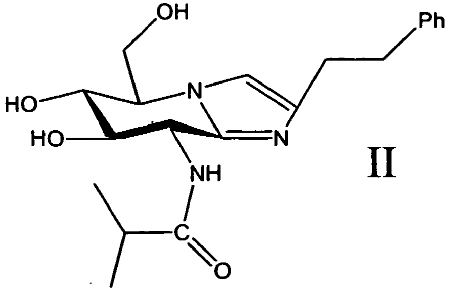

This compound has been shown to provide a high level of activity in the inhibition of a bacterial OGA (bOGA) with a selectivity of 100000 over inhibition of the hexosaminadases (HexA, HexB) and the compound is also active against human OGA (hOGA). Results of experiments using the compound of formula II are described hereafter together with results from other preferred compounds where -Y-Z is phenethyl, compounds III and IV below, where R¹ is methyl or ethyl. The known compound V (ref²⁰) the methyl ester of the naturally occuring gluco-nagstatin was studied at the same time for comparison purposes.

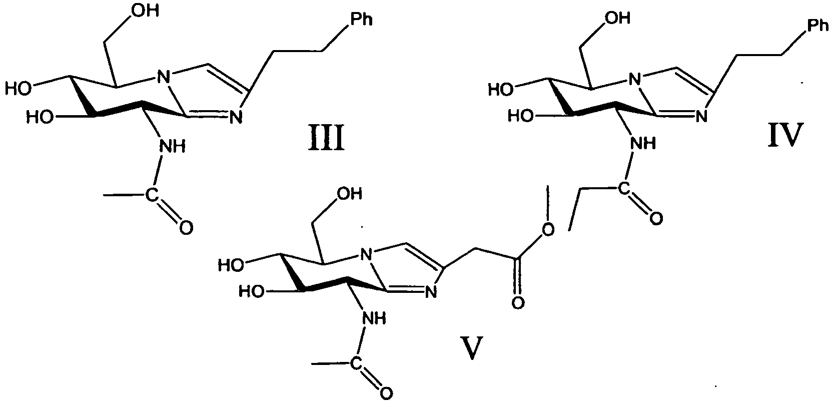

Thus the compounds of general formula I can be used to study the role of the O-GlcNAcase modification in human or animal cells. Furthermore the compounds of general formula I can have therapeutic use. The enzyme O-GlcNAcase has a role in the etiology of several diseases including type II diabetes, Alzheimers Disease, and cancer as discussed above. Compounds that achieve selective inhibition of O-GlcNAcase, avoiding or at least minimising inhibition of the lysomal β-hexosaminidases, can find therapeutic uses for the aforementioned disorders by inhibiting hyperphosphorylation, for example.

Thus according to a second aspect the present invention provides an in vitro method of inhibiting O-GlcNAcase enzymes comprising the step of administering a compound of general formula I to a cell or an extract containing O-GlcNAcase enzymes.

Thus according to a third aspect the present invention provides the use of a compound of general formula I in the preparation of a medicament for the treatment of Alzheimers Disease, diabetes or cancer.

The present invention further provides, in a fourth aspect, a method for the prevention or treatment of a condition mediated by the inhibition of O-GlcNAcase enzymes comprising the step of administering to an individual in need of such treatment a pharmaceutical composition comprising a compound of general formula I or a pharmaceutically acceptable salt thereof.

The present invention further provides, in a fifth aspect, a method for the prevention or treatment of Alzheimers disease, diabetes or cancer comprising the step of administering to an individual in need of such treatment a pharmaceutical composition comprising a compound of general formula I or a pharmaceutically acceptable salt thereof.

A small number of compounds of general formula I, including compound III, above, are known from WO2005/046611 (Amicus Therapeutics Inc), which describes a number of compounds for use in the treatment of Gaucher disease by acting as 'chaperones' for the mutant glucocerebrosidase associated with the disease. However, only compounds of formula I wherein n is 1, R¹ is methyl and -Y-Z is of certain particular forms are known from WO2005/046611.

Thus according to a yet further aspect the present invention provides compounds of general formula I: wherein n is 0 or 1;
X is hydrogen, halogen, -OH, -SH, NH₂; and
R¹ is a C₁ - C₆ saturated or unsaturated aliphatic chain which may be branched, a five membered, saturated or unsaturated ring, comprising from one to four nitrogen atoms or a substituted derivative thereof, 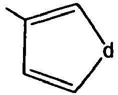 where d is CH₂, O or S; or, when n is 1, 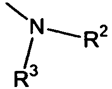 where R², R³ are independently hydrogen or C₁ - C₅ alkyl or are fused to form a saturated ring with the nitrogen which has from two to five carbon atoms;
-Y- is a linking group selected from the group consisting of -CH₂CH₂-, -CH=CH-, -C≡C-, -CH₂O-, -CH₂S- and -CH₂NH-; and
-Z is a hydrophobic group or an oligopeptide chain; with the provisos that,
when n is 1 and R¹ is methyl, -Y- is not -CH₂CH₂-, or-CH=CH-; and
when n is 1, R¹ is methyl and -Y- is a substituted or unsubstituted alkyl, alkenyl or alkynyl group of not more than two carbon atoms, -Z is -COOR⁴ wherein R⁴ is C₁ - C₆ saturated or unsaturated alkyl which may be branched, aryl, or heteroaryl, or -Z is an alkyl, alkenyl or alkynyl group of not more than two carbon atoms, or Z is an oligopeptide chain.

For use according to the present invention, the compounds described above or a physiologically acceptable salt, ester or other physiologically functional derivative thereof may be presented as a pharmaceutical formulation, comprising the compound or physiologically acceptable salt, ester or other physiologically functional derivative thereof, together with one or more pharmaceutically acceptable carriers therefore and optionally other therapeutic and/or prophylactic ingredients. The carrier(s) must be acceptable in the sense of being compatible with the other ingredients of the formulation and not deleterious to the recipient thereof.

Pharmaceutical formulations include those suitable for oral, topical (including dermal, buccal and sublingual), rectal or parenteral (including subcutaneous, intradermal, intramuscular and intravenous), nasal and pulmonary administration e.g., by inhalation. The formulation may, where appropriate, be conveniently presented in discrete dosage units and may be prepared by any of the methods well known in the art of pharmacy. All methods include the step of bringing into association an active compound with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of active compound. A tablet may be made by compression or moulding, optionally with one or more accessory ingredients. Compressed tablets may be prepared by compressing in a suitable machine an active compound in a free-flowing form such as a powder or granules optionally mixed with a binder, lubricant, inert diluent, lubricating agent, surface-active agent or dispersing agent. Moulded tablets may be made by moulding an active compound with an inert liquid diluent. Tablets may be optionally coated and, if uncoated, may optionally be scored. Capsules may be prepared by filling an active compound, either alone or in admixture with one or more accessory ingredients, into the capsule shells and then sealing them in the usual manner. Cachets are analogous to capsules wherein an active compound together with any accessory ingredient(s) is sealed in a rice paper envelope. An active compound may also be formulated as dispersable granules, which may for example be suspended in water before administration, or sprinkled on food. The granules may be packaged, e.g., in a sachet. Formulations suitable for oral administration wherein the carrier is a liquid may be presented as a solution or a suspension in an aqueous or non-aqueous liquid, or as an oil-in-water liquid emulsion.

Formulations for oral administration include controlled release dosage forms, e.g., tablets wherein an active compound is formulated in an appropriate release - controlling matrix, or is coated with a suitable release - controlling film. Such formulations may be particularly convenient for prophylactic use.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of an active compound with the softened or melted carrier(s) followed by chilling and shaping in moulds.

Pharmaceutical formulations suitable for parenteral administration include sterile solutions or suspensions of an active compound in aqueous or oleaginous vehicles.

Injectible preparations may be adapted for bolus injection or continuous infusion. Such preparations are conveniently presented in unit dose or multi-dose containers which are sealed after introduction of the formulation until required for use. Alternatively, an active compound may be in powder form which is constituted with a suitable vehicle, such as sterile, pyrogen-free water, before use.
An active compound may also be formulated as long-acting depot preparations, which may be administered by intramuscular injection or by implantation, e.g., subcutaneously or intramuscularly. Depot preparations may include, for example, suitable polymeric or hydrophobic materials, or ion-exchange resins. Such long-acting formulations are particularly convenient for prophylactic use.

Formulations suitable for pulmonary administration via the buccal cavity are presented such that particles containing an active compound and desirably having a diameter in the range of 0.5 to 7 microns are delivered in the bronchial tree of the recipient.

As one possibility such formulations are in the form of finely comminuted powders which may conveniently be presented either in a pierceable capsule, suitably of, for example, gelatin, for use in an inhalation device, or alternatively as a self-propelling formulation comprising an active compound, a suitable liquid or gaseous propellant and optionally other ingredients such as a surfactant and/or a solid diluent. Suitable liquid propellants include propane and the chlorofluorocarbons, and suitable gaseous propellants include carbon dioxide. Self-propelling formulations may also be employed wherein an active compound is dispensed in the form of droplets of solution or suspension.

Such self-propelling formulations are analogous to those known in the art and may be prepared by established procedures. Suitably they are presented in a container provided with either a manually-operable or automatically functioning valve having the desired spray characteristics; advantageously the valve is of a metered type delivering a fixed volume, for example, 25 to 100 microlitres, upon each operation thereof.

As a further possibility an active compound may be in the form of a solution or suspension for use in an atomizer or nebuliser whereby an accelerated airstream or ultrasonic agitation is employed to produce a fine droplet mist for inhalation.

Formulations suitable for nasal administration include preparations generally similar to those described above for pulmonary administration. When dispensed such formulations should desirably have a particle diameter in the range 10 to 200 microns to enable retention in the nasal cavity; this may be achieved by, as appropriate, use of a powder of a suitable particle size or choice of an appropriate valve. Other suitable formulations include coarse powders having a particle diameter in the range 20 to 500 microns, for administration by rapid inhalation through the nasal passage from a container held close up to the nose, and nasal drops comprising 0.2 to 5% w/v of an active compound in aqueous or oily solution or suspension.

It should be understood that in addition to the aforementioned carrier ingredients the pharmaceutical formulations described above may include, an appropriate one or more additional carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

Pharmaceutically acceptable carriers are well known to those skilled in the art and include, but are not limited to, 0.1 M and preferably 0.05 M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

Formulations suitable for topical formulation may be provided for example as gels, creams or ointments. Such preparations may be applied e.g. to a wound or ulcer either directly spread upon the surface of the wound or ulcer or carried on a suitable support such as a bandage, gauze, mesh or the like which may be applied to and over the area to be treated.

Liquid or powder formulations may also be provided which can be sprayed or sprinkled directly onto the site to be treated, e.g. a wound or ulcer. Alternatively, a carrier such as a bandage, gauze, mesh or the like can be sprayed or sprinkle with the formulation and then applied to the site to be treated.

Therapeutic formulations for veterinary use may conveniently be in either powder or liquid concentrate form. In accordance with standard veterinary formulation practice, conventional water soluble excipients, such as lactose or sucrose, may be incorporated in the powders to improve their physical properties. Thus particularly suitable powders of this invention comprise 50 to 100% w/w and preferably 60 to 80% w/w of the active ingredient(s) and 0 to 50% w/w and preferably 20 to 40% w/w of conventional veterinary excipients. These powders may either be added to animal feedstuffs, for example by way of an intermediate premix, or diluted in animal drinking water.

Liquid concentrates of this invention suitably contain the compound or a derivative or salt thereof and may optionally include a veterinarily acceptable water-miscible solvent, for example polyethylene glycol, propylene glycol, glycerol, glycerol formal or such a solvent mixed with up to 30% v/v of ethanol. The liquid concentrates may be administered to the drinking water of animals.

The present invention also provides a method for the synthesis of compounds according to general formula VI: 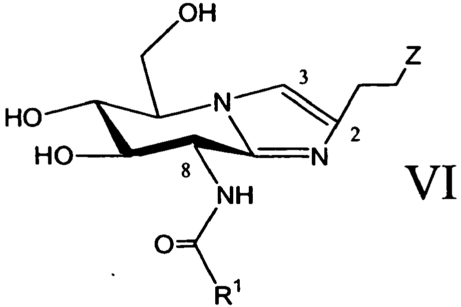 where Z and R¹ have the same meaning as before, comprising the steps of :
a) reacting a compound of formula VII : 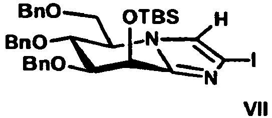 with a compound of formula VIII 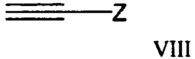 in the presence of a palladium catalyst such as Pd(PPh₃)₄ and then removing the TBS protecting group with TBAF **tetrabutylammonium fluoride** to afford a compound of formula IX: 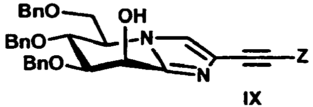
b) reacting the compound of formula IX with DPPA (diphenylphosphorylazide),DBU (1,8-diazabicyclo[5.4.0]undec-7-ene)to form the azido compound X; 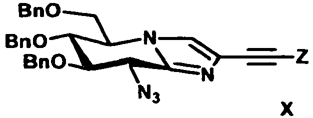
c) reducing the azide with PPh₃ to the amine and acylating with a compound of formula XI (R₁CO)₂O, to give a compound of formula XII: 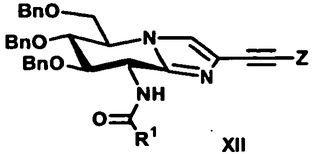 ;and
d) saturating the triple bond and removing the benzyl protecting groups to afford the compounds of formula VI.

Similarly, other compounds of general formula I can be synthesised by a similar synthetic route with appropriate modifications to the starting material and reagents employed.

Further preferred features and advantages of the present invention will appear from the following detailed description of embodiments illustrated with reference to the accompanying drawings in which:
Fig 1. shows a method of synthesis of compounds of the invention;
Figs. 2 and 3 show graphically the results of kinetic studies of enzyme inhibition using a compound of the invention;
Figs. 4A, 4B show photographs of immunoblot detection of O-GlcNAc modifications on cellular proteins.; and
Fig 5 shows graphically a comparison of the inhibition of human OGA (hOGA) by Compound II and PUGNAc

### Description of Some Preferred Embodiments and Experimental

### Results

### Example 1 Preparation of Compound II

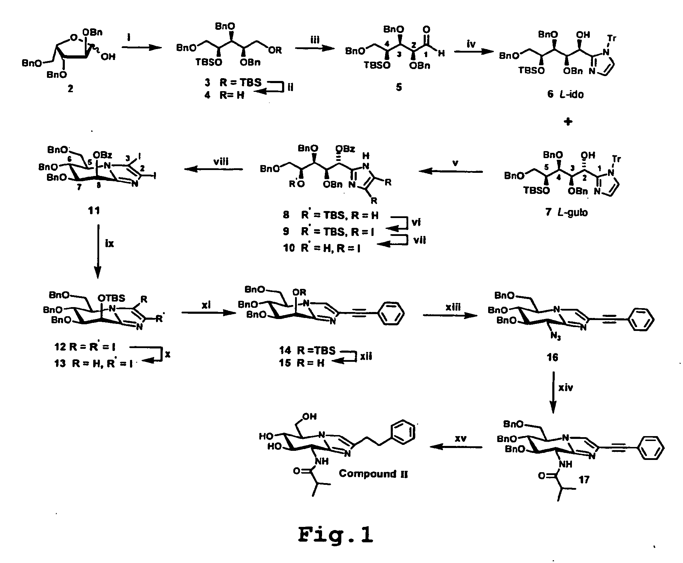

The reaction scheme shown in Figure 1 illustrates the synthetic approach to compound II of the invention as an example of the general synthetic approach.
The reagents and conditions for each step shown in the figure are summarised below, followed by a more detailed description of the synthesis. Compounds III and IV were prepared by means of the same synthetic route but the isobutyric anhydride, (*i*-PrCO)₂O, utilised as acylating agent at step xiv (below) was replaced by acetic anhydride (for III) and propionic anhydride (for IV) respectively. The known compound V, used for comparative purposes was prepared by analogy with the published procedure ¹⁵.

*Reagents and conditions*: (i) a) NaBH₄, MeOH, 0° to RT, 1h; b) TBSCl, ImH, DMF, +55°C, overnight, 95% overall; (ii) TFA, H₂O, CHCl₃, 15 min, RT, 65%; (iii) (COCl)₂, DMSO, Et₃N, DCM, -60°C, 93%; (iv) N-trityl imidazole, BuLi, THF, -78°C, 70% of a 3:1 mixture of **7** and **6**; (v) a) TFA, DCM then Et₃SiH, 25 min, RT; b) BzCl, Py, DMAP cat., 30 min, RT, 91% overall; (vi) *N*-Iodosuccinimide, MeCN, 6h, RT, 90%; (vii) HCl, 1,4-dioxane, 2h , RT, quant.; (viii) Tf₂O, Py, DCM,-15°C to RT, 12 h then +50°C, 1h, 89%; ix) (a) MeONa, MeOH, DCM, 10 min, RT; (b) TBSOTf, i-Pr₂NEt, DCM, -15°C to RT; 91% overall; x) EtMgBr, THF, 20 min, 0°, 93%; xi) C₆H₅CCH, Pd(PPh₃)₄, CuI, Et₃N, DMF, 12h, +80°C, 96%; xii) TBAF, THF, 1h, RT, 83%; xiii) DPPA, DBU, toluene-THF, 12h, RT, 90%; xiv) PPh₃, THF-H₂O, 3h, +60°C, then (*i*-PrCO)₂O, Et₃N, 1h RT, 95%; xv) Pd(OH)₂ , H₂ 14.5 psi, AcOH, 5h RT, 60%.

### Detailed Experimental Procedure

### General

All reactions were performed in oven-dried glassware under an inert atmosphere (argon) unless noted otherwise. Dichloromethane, toluene and acetonitrile were distilled from CaH₂ prior to use. Tetrahydrofuran was distilled from sodium-benzophenone prior to use. N,N-dimethylformamide, pyridine, triethylamine were anhydrous grade solvents from Fluka. Ethyl acetate (EA), petroleum spirit 40-60° (PE), diethyl ether (EE), and chloroform were laboratory grade solvents. Analytical thin layer chromatography (TLC) was performed on Merck silica gel 60 F254 aluminium plates (0.25mm). Compounds were visualized by UV light, or by dipping the plate into acidic potassium permanganate aqueous solution followed by washing out the excess of the reagent, or by charring the plate at *ca.* 300°C after dipping in one of the following solutions: 15% sulphuric acid in water-ethanol, 5% phosphomolybdic acid in ethanol, orcinol in acidic ethanol. Flash column chromatography was performed on Merck Kieselgel 60 (230-400 mesh). NMR spectra were recorded on a Bruker DPX300 or Bruker AVANCE II 500 spectrometer in CDCl₃ unless otherwise stated. Chemical shifts are referenced to internal CDCl₃ (7.26 ppm ¹H, 77.0 ppm ¹³C). Splitting patterns of spectral multiplets are indicated as s, singlet; d, doublet; bs broad singlet; bd broad dublet; t triplet; quint quintet for ¹H NMR data. Signals were assigned by means of DEPT, COSY, HSQC, and HMBC spectra. High-resolution mass spectra (HRMS) were obtained on a microTOF Bruker Daltonics instrument. Optical rotations were measured in chloroform on a Perkin Elmer 343 polarimeter.

**Compound 3.** To a solution of **2** ^{21,22} (6.25 g, 14.9 mmol) in MeOH (100 ml) sodium borohydride (NaBH₄; 0.56 g, 14 .8 mmol) was added at 0°C (ice-bath). The resulting slurry was stirred for 1h while the reaction was allowed to warm up to the RT. The remaining sodium borohydride was quenched by careful addition of glacial acetic acid (3.4 ml, 59.24 mmol) and the reaction was evaporated to dryness. The residue was dissolved in DCM and washed with a mixture of saturated NaHCO₃ solution and brine. The aqueous layer was extracted with DCM once more. The combined organic layer was dried and evaporated. The residue was dissolved in DMF (90 ml) and treated with *tert*-butyl(chloro)dimethylsilane (TBSCl; 5.42 g, 36 mmol) in the presence of imidazole (ImH; 4.9 g, 72 mmol) for 16h at +50°C. The reaction was cooled down and quenched by the addition of MeOH (5 ml). The bulk of DMF was removed in vacuum. The residue was dissolved in ethyl acetate and washed with water. The aqueous layer was extracted with ethyl acetate once more. The combined organic layer was dried and evaporated. The residue was purified by flash column chromatography in PE-EE gradient 1→10% to give 9.24 g (14.2 mmol, 95%) of the target product.
[α]_{D} = -21.1° c 1.0 CHCl₃
HRMS: Positive mode, m/z = 673.3766; expected for C₃₈H₅₈NaO₅Si₂ [M+Na]⁺ = 673.3720.
δ_{H} (500 MHz): -0.01 and 0.002 and 0.04 (12H, 4×s,-Si(CH₃)C(CH₃)₃), 0.88 (18H, 2×s, -Si(CH₃)C(C*H*₃)₃), 3.43 (1H, dd, *J*_{4,5} = 7.2 Hz, *J*_{5a,5b} = 10.3 Hz, H-5a), 3.6 (1H, dd, *J*_{1,2} = 5.6 Hz, *J*_{1a,1b} = 10 Hz, H-1a), 3.64 (1H, dd, *J* = 5.6 Hz, *J* = 3.2 Hz, H-3), 3.68-3.75 (3H, m), 4.02 (1H, m, H-4), 4.43 (2H, AB spectrum, *J_{gem}* = 12 Hz, PhC*H*₂O-), 4.54 and 4.63 (2H, AB spectrum, *J_{gem}* = 12 Hz, PhC*H*₂O-), 4.65 (2H, AB spectrum, *J_{gem}* = 11.5 Hz, PhC*H*₂O-), 7.23 -7.33 (15H, m, 3×*Ph*C*H*₂O-).
δ_{C} (125 MHz): -5.4, -5.3, -4.8, -4.3, 18.2, 18.24, 26, 62.4, 72.18, 72.5, 73.1, 73.4, 74.1, 76.9, 77.1, 77.4, 78.9, 127.4, 127.6, 127.6, 128.3, 138.7.

**Compound 4:** To a solution of **3** (4.4 g, 6.76 mmol) in chloroform (70 ml) aqueous trifluoroacetic acid (TFA-H₂O 10:1 v/v; 7 ml) was added at RT. The resulting solution was kept for 10 min, diluted with toluene (70 ml) and evaporated to dryness. The residue was dissolved in DCM and washed with a mixture of saturated NaHCO₃ solution and brine. The aqueous layer was extracted with DCM once more. The combined organic layer was dried and evaporated. The residue was purified by flash column chromatography in PE-EE 10% and then in PE-EA gradient 10 →20% to give 1.16 g (1.78 mmol, 26%) of the unchanged starting material and 2.24 g (4.17mmol, 62%) of the target product (83% based on the recovered starting material).
[α]_{D} = -8.5° c 1.1 CHCl₃
HRMS: Positive mode, m/z = 559.2902; expected for C₃₂H₄₄NaO₅Si [M+Na]⁺ = 559.2856.
δ_{H} (300 MHz): 0.04 and 0.0 (6H, 2xs, -Si(C*H*₃)C(CH₃)₃), 0.82 (9H, s, -Si(CH₃)C(C*H*₃)₃), 2.52 (1H, t, *J* = 6.5 Hz, 1-OH), 3.48 (1H, dd, *J*_{4,5} = 7 Hz, *J*_{5a,5b} = 10 Hz, H-5a), 3.5-3.7 (5H, m), 4 (1H, ddd, *J* = 3 Hz, *J* = 4.3 Hz, H-4), 4.42 (2H, s, PhC*H*₂O-), 4.55 and 4.62 (2H, AB spectrum, *J_{gem}* = 11.5 Hz, PhC*H*₂O-), 4.61 and 4.68 (2H, AB spectrum, *J_{gem}* = 11.7 Hz, PhC*H*₂O-), 7.2 -7.3(15H, m, 3×*Ph*C*H*₂O-).
δ_{C} (75 MHz) : -4.9, -4.4, 18.15, 25.9, 62.24, 71.7, 72.4, 73.3, 73.4, 74.2, 79.9, 81.2, 127.5, 127.6, 127.7, 127.8, 128.1, 128.16, 128.33, 128.44, 138.4, 138.45.

**Compound 5:** To a solution of oxalyl chloride (1.12 ml, 12.9 ml) in DCM (70 ml) a solution of dimethyl sulfoxide (1.83 ml, 12.9 mmol) in DCM (3ml) was added dropwise at -60°C. The resulting solution was stirred for 15 min, and then a solution of **4** (4.62 g, 8.6 mmol) in DCM (20ml) was added via cannula. The resulting solution was stirred at -60°C for 1h and then triethylamine (5.4 ml, 38.7 mmol) was added dropwise. The resulting slurry was stirred for 10 min and then warmed up to 0°C and quenched by the addition of 1M HCl solution (40 ml). The layers were separated and the aqueous layer was extracted with DCM once more. The combined organic layer was washed successively with water and a mixture of saturated NaHCO₃ solution and brine, dried and evaporated. The residue was purified by flash column chromatography in PE-EA 10 % to give 4.27 g (8 mmol, 93%) of the target product.
[α]_{D} = -16.3° c 1.04 CHCl₃
HRMS: Positive mode, m/z = 535.2854; expected for C₃₂H₄₃O₅Si [M+H]⁺ = 535.2880.
δ_{H} (500 MHz): 0.0 and 0.04 (6H, 2×s, -Si(C*H*₃)C(CH₃)₃), 0.89 (9H, s, -Si(CH₃)C(C*H*₃)₃), 3.5 (1H, dd, *J*_{5a,4} = 7 Hz, *J*_{5a,5b} = 10 Hz, H-5a), 3.74 (1H, dd, *J*_{5b,4} = 7 Hz, H-5b), 3.84 (1H, dd, *J*_{3.4} = 7 Hz, *J*_{3,2} = 10 Hz, H-3), 4.02 (1H, d, H-2), 4.05 (1H, ddd, H-4), 4.47 (2H, s, PhC*H*₂O-), 4.52 and 4.68 (2H, AB spectrum, *J_{gem}* = 11.5 Hz, PhC*H*₂O-), 4.61(2H, s, PhC*H*₂O-), 7.2 -7.35 (15H, m, 3×*Ph*C*H*₂O-), 9.65 (1H, s, H-1).
δ_{C} (125 MHz): -4.8, -4.5, 18.1, 25.9, 71.5 (C-4 and C-5), 73.2, 74.1, 80.2 (C-3), 83.2 (C-2), 127.6, 127.7, 128, 128.1, 128.2, 128.4, 128.44, 128.5, 137.2, 137.8, 138.3, 202.3 (C-1).

**Compounds 6 and 7:** To a solution of tritylimidazole (2.67 g, 8.6 mmol) in THF (85 ml) cooled to -20°C a stock 1.6 M solution n-butyl lithium in hexanes (5.13 ml, 8.2 mmol) was added dropwise. The resulting wine red coloured solution was allowed to warm up to RT and stirred for 30 min. The solution was then cooled to -78°C and a solution of aldehyde (4.17 g, 7.8 mmol) in THF (15ml) was added dropwise. The resulting solution was stirred for 30 min and then allowed to warm to RT in 30 min. The reaction was quenched by addition of ammonium chloride solution and extracted with ethyl acetate. The aqueous layer was extracted with ethyl acetate once more. The combined organic layer was dried and evaporated. The residue was purified by flash column chromatography in PE-EA gradient 10→20→30% to give 1.18 g (1.4 mmol, 18%) of **6** (R_{f} = 0.55 PE-EA 20%) and 3.48 g (4.13 mmol, 53%) of **7** (R_{f} = 0.4 PE-EA 20%).
**6:** [α]_{D} = -10.5° *c* 1.24 CHCl₃
HRMS: Positive mode, m/z = 845.4344; expected for C₅₄H₆₁N₂O₅Si [M+H]⁺ = 845.4350.
δ_{H} (500 MHz): 0.0 and 0.02 (6H, 2×s, -Si(C*H*₃)C(CH₃)₃), 0.89 (9H, s, -Si(CH₃)C(C*H*₃)₃), 2.94 (1H, dd, *J*_{4,5} = 6.5 Hz, *J*_{4,3} = 4.1 Hz, H-4), 3.1 (1H, bs, 2-OH), 3.14 (1H, dd, *J*_{6a,5} = 5.4, *J*_{6a,6b} = 10.2 Hz, H-6a), 3.37 (1H, dd, *J*_{6b,5} = 3 Hz, H-6b), 3.8 (1H, dd, *J*_{3,2} = 3.2 Hz, H-3), 3.93 (1H, ddd, H-5), 3.97 and 4.41 (2H, AB spectrum, *J_{gem}* = 11.5 Hz, PhC*H*₂O-), 4.3 (1H, bs, H-2), 4.33 (2H, s, PhC*H*₂O-), 4.71 and 4.93 (2H, AB spectrum, *J_{gem}* = 11.2 Hz, PhC*H*₂O-), 6.78 (1H, m), 7.02 (2H, m), 7.13-7.4 (28H, m).
δ_{C} (125 MHz): -4.4, 18.2, 26.1, 66.6 (C-2), 72.8 (C-6), 72.9 (C-5), 73.2, 73.8, 74.4, 75.3, 78.8 (C-3), 81.7 (C-4), 122.2, 126.08, 127.06, 127.3, 127.4, 127.8, 128.1, 128.3, 128.5, 130, 138.6, 138.8, 138.9, 142.9, 150.7.
**7:** [α]_{D} = -67.5° c 1.34 CHCl₃
HRMS: Positive mode, m/z = 845.4344; expected for C₅₄H₆₁N₂O₅Si [M+H]⁺ = 845.4350.
δ_{H} (500 MHz): -0.09 and 0.0 (6H, 2×s, -Si(C*H*₃)C(CH₃)₃), 0.82 (9H, s, -Si(CH₃)C(C*H*₃)₃), 3.52 (1H, dd, *J*_{6a,5} = 6.5 Hz, *J*_{6a,6b} = 10.4 Hz, H-6a), 3.73 (1H, dd, *J*_{4,5} = 7.3, *J*_{4,3} = 2.6 Hz, H-4), 3.78 (1H, dd, *J*_{6b,5} = 2.2 Hz, H-6b), 4.12 (1H, ddd, *J*_{5,4} = 6.7 Hz, H-5), 4.24 (1H, dd, *J*_{3,2} = 8.7 Hz, H-3), 4.27 and 4.57 (2H, AB spectrum, *J_{gem}* = 12.4 Hz, PhC*H*₂O-), 4.41 (2H, s, PhC*H*₂O-), 4.48 and 4.6 (2H, AB spectrum, *J_{gem}* = 10.7 Hz, PhC*H*₂O-), 4.5 (1H, dd, *J*_{2,OH} = 4 Hz, H-2), 6.79 (1H, m), 6.85-7.4 (31H, m).
δ_{C} (125 MHz): -4.7, 18.2, 26, 65.9 (C-2), 72.6 (C-5), 73.1, 73.2 (C-6), 73.8, 75.5, 79.6 (C-4), 80.13 (C-3), 122.3, 125.9, 126.17, 126.5, 127, 127.2, 127.6, 127.8, 127.9, 128.1, 128.4, 128.6, 130, 130.2, 138.7, 139, 139.3, 141, 142.5, 150.2 (C-1).

**Compound 8:** To a solution of **7** (1.13 g, 1.34 mmol) in chloroform (CHCl₃; 35 ml) trifluoroacetic acid (11 ml) was added at RT to give bright yellow solution. The reaction was kept for 25 min at RT and quenched by addition of an excess of triethylsilane (2ml, 12.5 mmol) (yellow colouration disappeared). The reaction was diluted with toluene (50 ml) and evaporated. The residue was taken up into DCM and washed with a mixture of NaHCO₃ solution and brine. The aqueous layer was extracted with DCM once more. The combined organic layer was dried and evaporated. The residue was dissolved in pyridine (15ml) and treated with benzoyl chloride (3 mmol) in the presence of catalytic amount of 4-*N,N*-dimethylaminopyridine. After 30 min the reaction was quenched by addition of water and stirred for 20 min. The bulk of pyridine was evaporated in vacuum. The residue was dissolved in a mixture of methanol and triethylamine 4:1 (10 ml), kept for 30 min at RT and evaporated. The residue was purified by flash column chromatography in PE-EA gradient 10→20→25% to give 0.86 g (1.21 mmol, 91%) of the target product.
[α]_{D} = -3.9° c 0.87 CHCl₃
HRMS: Positive mode, m/z = 707.3481; expected for C₄₂H₅₁N₂O₆Si [M+H]⁺ = 707.3516.
δ_{H} (500 MHz): 0.0 (6H, s, -Si(C*H*₃)C(CH₃)₃), 0.82 (9H, s,-Si(CH₃)C(C*H*₃)₃), 3.43 (1H, dd, *J*_{6a,5} = 6.5 Hz, *J*_{6a,6b} = 10.1 Hz, H-6a); 3.6 (1H, dd, *J*_{4,3} = 5 Hz, *J*_{4,5} = 4.3 Hz, H-4), 3.65 (1H, dd, *J*_{6b,5} = 3.1 Hz, H-6b), 4.1 (1H, ddd, H-5), 4.33 and 4.43 (2H, AB spectrum, *J*_{gem} = 11.6 Hz, PhC*H*₂O-), 4.36 (2H, s, PhC*H*₂O-), 4.39 and 4.46 (2H, AB spectrum, *J*_{gem} = 10.8 Hz, PhC*H*₂O-), 4.47 (1H, dd, *J*_{3,2} = 5.3 Hz, H-3), 6.16 (1H, d, H-2), 6.83 (1H, s, H-3'), 6.96-7.25 (16H, m, 3×*Ph*CH₂O-, H-2'), 7.3 (2H, m), 7.46 (1H, m), 7.9 (2H, m), 9.7 (1H, bs, NH).
δ_{C} (125 MHz): -4.8, -4.3, 18.2, 26, 69.9 (C-2), 71.7 (C-5), 72.15 (C-6), 73.1, 73.9, 74.9, 79.18 (C-3/C-4), 79.25 (C-3/C-4), 116.3, 127.4, 127.6, 127.8, 127.9, 128.4, 128.5, 129.6, 129.9, 133.2, 137.7, 137.8, 138.5, 144 (C-1), 165.3.

**Compound 9:** To a stirred solution of **8** (1.03 g, 1.46 mmol) in acetonitrile (15 ml) N-iodosuccinimide (0.818 g, 3.65 mmol) was added at RT. The reaction was further stirred for 12h in the dark. The reaction was diluted with DCM and washed with a mixture of 0.5M sodium thiosulphate solution and brine. The aqueous layer was extracted with DCM once more. The combined organic layer was dried and evaporated. The residue was purified by flash column chromatography in PE-EA gradient 5→10→15% to give 1.26 g (1.31 mmol, 90%) of the target product.
[α]_{D} = -5.6° c 1.19 CHCl₃
HRMS: Positive mode, m/z = 959.1440; expected for C₄₂H₄₉I₂N₂O₆Si [M+H]⁺ = 959.1449.
δ_{H} (500 MHz): 0.0 and 0.02 (6H, 2×s, -Si(C*H*₃)C(CH₃)₃), 0.83 (9H, s, -Si(CH₃)C(C*H*₃)₃), 3.43 (1H, dd, *J*_{6a,5} = 6.7 Hz, *J*_{6a,6b} = 10.3 Hz, H-6a); 3.64 (1H, dd, *J*_{4,3} = 3 Hz, *J*_{4,5} = 6 Hz, H-4), 3.67 (1H, dd, *J*_{6b,5} = 2.6 Hz, H-6b), 4.12 (1H, ddd, H-5), 4.3 and 4.5 (2H, AB spectrum, *J*_{gem} = 11 Hz, PhC*H*₂O-), 4.38 and 4.43 (2H, AB spectrum, *J*_{gem} = 12.3 Hz, PhC*H*₂O-), 4.45 (2H, AB spectrum, PhC*H*₂O-), 4.41 (1H, H-3), 6.11 (1H, d, *J*_{2,3} = 4.5, Hz, H-2), 7.0 (4H, m), 7.1-7.26 (11H, m), 7.33 (2H, m), 7.5 (1H, m), 7.9 (2H, m), 9.7 (1H, bs, NH).
δ_{C} (125 MHz): -4.7, -4.4, 18.2, 26, 70 (C-2), 71.7 (C-5), 72 (C-6), 73.2, 74, 74.9, 75.8, 79.1 (C-3), 79.6 (C-4), 95.1, 127.5, 127.7, 127.9, 128.1, 128.2, 128.4, 128.5, 128.6, 129.3, 130, 133.5, 137.3, 137.4, 138.4, 150 (C-1), 165.2.

**Compound 10:** To intensively stirred solution of **9** (1.24 g, 1.3 mmol) in 1,4-dioxane (15 ml) concentrated hydrochloric acid (1.2 ml) was added at RT. The reaction was further stirred for 2h, diluted with DCM and washed successively with water and a mixture of NaHCO₃ solution and brine. The aqueous layer was extracted with DCM once more. The combined organic layer was dried and evaporated. The residue was purified by flash column chromatography in PE-EA gradient 10→20→30% to give 1.06 g (1.26 mmol, 96%) of the target product.
[α]_{D} = -6.7° c 0.94 CHCl₃
HRMS: Positive mode, m/z = 845.0585; expected for C₃₆H₃₅I₂N₂O₆ [M+H]⁺ = 845.0585.
δ_{H} (300 MHz): 3.18 (1H, d, *J*_{5,OH} = 6 Hz, 5-OH); 3.56 (2H, m, H-6a, H-6b), 3.85 (1H, dd, *J*_{4,3} = 6 Hz, *J*_{4,5} = 3 Hz, H-4), 4.15 (1H, m, H-5), 4.5 (1H, dd, H-3), 4.51 (2H, AB spectrum, PhC*H*₂O-), 4.55 and 4.64 (2H, AB spectrum, *J*_{gem} = 11 Hz, PhC*H*₂O-), 4.68 (2H, AB spectrum, PhC*H*₂O-), 6.3 (1H, d, *J*_{2,3} = 4 Hz, H-2), 7.16-7.42 (17H, m,), 7.58 (1H, m), 8 (2H, m), 10.1 (1H, bs, NH).
δ_{C} (75 MHz) : 69.5 (C-5), 69.7 (C-2), 70.8 (C-6), 73.3, 74.9, 75, 76.2, 78.4 (C-4), 80 (C-3), 94.9, 127.7, 127.8, 127.9, 128.1, 128.2, 128.4, 128.6, 129.1, 130, 133.6, 137.4, 137.6, 137.9, 149.9 (C-1), 165.7.

**Compound 11:** To a solution of **10** (1.04 g, 1.23 mmol) and Py (0.41ml, 5.05 mmol) in DCM (15 ml) cooled to -15°C trifluoromethanesulfonic anhydride (0.62ml, 3.77mmol) was added dropwise. The reaction was allowed to warm-up to the RT and left stirred for 24 h. At this point TLC PE-EA 15% still showed the presence of the upper spot (presumably intermediate triflate) and the more polar major product. The reaction was heated for 1.5 h at 50°C to promote the disappearance of the upper spot. The reaction was cooled down, diluted with DCM and washed with a mixture of NaHCO₃ solution and brine. The aqueous layer was extracted with DCM once more. The combined organic layer was dried evaporated. The residue was purified by flash column chromatography in PE-EA gradient 10→20→30% to give 0.902 g (1.1 mmol, 89%) of the target product.
[α]_{D} = -101.6° c 1.0 CHCl₃
HRMS: Positive mode, m/z = 827.0454; expected for C₃₅H₃₃I₂N₂O₅ [M+H]⁺ = 827.0479.
δ_{H} (500 MHz): 3.74 (1H, m, *J*_{5*a,5} = 3.6 Hz, *J*_{5*a,5*b} = 9.8 Hz, H-5*a), 4.0 (1H, m, *J*_{5*b,5} = 7.4 Hz, H-5*b); 4.1 (1H, dd, *J*_{7,6} = 7.5 Hz, *J*_{7,8} = 3.6 Hz, H-7), 4.3 (1H, ddd, *J*_{5,6} = 3.6 Hz, H-5), 4.46 (2H, AB spectrum, *J*_{gem} = 12.1 Hz, PhC*H*₂O-), 4.54 and 4.75 (2H, AB spectrum, *J*_{gem} = 11.7 Hz, PhC*H*₂O-), 4.56 (1H, dd, H-6), 4.64 and 4.76 (2H, AB spectrum, *J*_{gem} = 11.5 Hz, PhC*H*₂O-), 6.65 (1H, d, H-8), 7.16-7.35 (15H, m), 7.38 (2H, m), 7.55 (1H, m), 8.06 (2H, m).
δ_{C} (125 MHz): 61.7 (C-5), 63.9 (C-8), 68.9 (C-5*), 72.4, 73.2, 73.8, 74 (C-6), 77.1 (C-7), 81.2 (C-2), 97.8 (C-3), 127.7, 128.02, 128.08, 128.22, 128.3, 128.4, 128.6, 129.6,130.1, 133.3,137.1,137.4, 137.5, 146.7 (C-8a), 165.25.

**Compound 12:** To a solution of **11** (0.885 g, 1.07mmol) in DCM (15 ml) stock 25% solution of sodium methoxide (0.15 ml) was added at RT. After 10 min the reaction was quenched by addition of dry ice. After the reaction was attained RT it was diluted with DCM and washed with brine. The aqueous layer was extracted once more with DCM. The combined organic layer was dried and evaporated to dryness. The residue was dissolved in anhydrous DCM (12 ml) and treated with *tert*-butyldimethylsilyl trifluoromethanesulfonate (0.3 ml, 1.3 mmol) in the presence of disopropylethylamine (0.3ml, 1.7mmol) at 0°C (ice bath). The reaction was allowed to warm-up to the RT and left stirred for 30 min. The reaction was quenched with MeOH (1 ml) diluted with DCM and washed with a mixture of NaHCO₃ solution and brine. The aqueous layer was extracted with DCM once more. The combined organic layer was dried and evaporated. The residue was purified by flash column chromatography in PE-EE gradient 10→20% to give 0.822 g (0.98 mmol, 91%) of the target product.
[α]_{D} = -82.8° c 0.83 CHCl₃
HRMS: Positve mode, m/z = 837.1047; expected for C₃₅H₄₃I₂N₂O₄Si [M+H]⁺ = 837.1082.
δ_{H} (500 MHz): 0.0 and 0.13 (6H, 2×s, -Si(C*H*₃)C(CH₃)₃), 0.83 (9H, s, -Si(CH₃)C(C*H*₃)₃), 3.6 (1H, m, , *J*_{5*a,5} = 4.9 Hz, *J*_{5*a,5*b} = 9.2 Hz, H-5*a), 3.62 (1H, dd, *J*_{7,6} = 7.6 Hz, *J*_{7,8} = 2.6 Hz, H-7), 3.73 (1H, dd, *J*_{5*b,5} = 9.2 Hz, H-5*b), 4.3 (1H, ddd, *J*_{5,6} = 2.5 Hz, H-5), 4.36 (1H, dd, H-6), 4.5 (2H, AB spectrum, PhC*H*₂O-), 4.58 and 4.72 (2H, AB spectrum, *J*_{gem} = 12 Hz, PhC*H*₂O-), 4.69 and 4.74 (2H, AB spectrum, *J*_{gem} = 11 Hz, PhC*H*₂O-), 4.97 (1H, d, H-2), 7.2-7.33 (15H, m).
δ_{C} (125MHz): -4.9, -4.6, 18.2, 25.8, 62.5 (C-5), 64.5 (C-8), 70.8 (C-5*), 72.2, 73.2, 73.4, 77.8 (C-6), 81.3 (C-7), 81.5 (C-2), 95.9 (C-3), 127.8, 127.9, 128, 128.4, 128.6, 137.6, 137.8, 138.1, 150.7 (C-8a).

**Compound 13:** To a solution of **12** (0.8 g, 0.956 mmol) in THF (10 ml) ethyl magnesium bromide solution in THF (1.3 ml, 1.3 mmol) was added at 0°C. After 10 min the reaction was quenched by addition of saturated ammonium chloride solution. The reaction was diluted with ethyl acetate and washed with brine. The aqueous layer was extracted once more with ethyl acetate. The combined organic layer was dried and evaporated. The residue was purified by flash column chromatography in PE-EA 15% to give 0.632 g (0.89 mmol, 93%) of the target product.
HRMS: Positive mode, m/z = 711.2088; expected for C₃₅H₄₄IN₂O₄Si [M+H]⁺ = 711.2115.
δ_{H} (500 MHz): 0.0 and 0.23 (6H, 2xs, -Si(C*H*₃)C(CH₃)₃), 0.89 (9H, s, -Si(CH₃)C(C*H*₃)₃), 3.5 (1H, dd, *J*_{5a*,5} = 7 Hz, *J*_{5a*,5b*} = 10 Hz, H-5*a), 3.62 (1H, dd, *J*_{5b*,5} = 3 Hz, H-5*b), 3.74 (1H, dd, *J*_{7,8} = 2.3 Hz, *J*_{7,6} = 9. 3 Hz, H-7), 4.05 (1H, ddd, *J*_{5,6} = 10 Hz, H-5), 4.2 (1H, dd, H-6), 4.42 (2H, AB spectrum, PhC*H*₂O-), 4.62 and 4.92 (2H, AB spectrum, *J*_{gem} = 11.6 Hz, PhC*H*₂O-), 4.64 and 4.8 (2H, AB spectrum, *J*_{gem} = 11.8 Hz, PhC*H*₂O-), 5.07 (1H, d, H-8), 7.14 (1H, s, H-3), 7.19-7.5 (15H, m, 3×*Ph*CH₂O-).
δ_{C} (125 MHz): -4.9, -4.5, 18.3, 25.9, 60 (C-5), 63.6 (C-8), 70.5 (C-5*), 71.8, 73.2 (C-6), 74.8, 80.9 (C-7), 82 (C-2), 124.5 (C-3), 127.83, 128, 128.5, 128.6, 137.4, 137.8, 137.9, 147.2 (C-8a).

**Compound 14:** A solution of **13** (0.315 g, 0.44 mmol), phenyl acetylene (0.243 ml, 2.22 mmol) and triethylamine (0.31ml, 2.22 mmol) in DMF (5 ml) was degassed by freezing, evacuating and filling with argon three times. Cuprous iodide (0.01 g, 0.05 mmol) and Pd catalyst (0.058 g, 0.05 mmol) were then added to the degassed solution. The reaction was stirred for 16 h at +80°C. The reaction was concentrated in vacuum. The residue was dissolved in ethyl acetate and washed with water and brine. The aqueous layer was extracted with ethyl acetate once more. The combined organic layer was dried and evaporated. The brown residue was purified by flash column chromatography in PE-EA 15% to give 0.29 g (0.42 mmol, 96 %) of the target product as an amber coloured amorphous compound.
HRMS: Positive mode, m/z = 685.3408; expected for C₄₃H₄₉N₂O₄Si [M+H]⁺ = 685.3462.
δ_{H} (500 MHz): 0.0 and 0.22 (6H, 2×s, -Si(C*H*₃)C(CH₃)₃), 0.85 (9H, s, -Si(CH₃)C(C*H*₃)₃), 3.51 (1H, dd, *J*_{5a*,5} = 7.4 Hz, *J*_{5a*,5b*} = 10 Hz, H-5*a), 3.62 (1H, dd, *J*_{5b*,5} = 3.1 Hz, H-5*b), 3.76 (1H, dd, *J*_{7,8} = 2 Hz, *J*_{7,6} = 9.1 Hz, H-7), 4.07 (1H, ddd, *J*_{5,6} = 10 Hz, H-5), 4.21 (1H, dd, H-6), 4.43 (2H, AB spectrum, PhC*H*₂O-), 4.62 and 4.92 (2H, AB spectrum, *J*_{gem} = 11.6 Hz, PhC*H*₂O-), 4.65 and 4.8 (2H, AB spectrum, *J*_{gem} = 11.8 Hz, PhC*H*₂O-), 5.08 (1H, d, H-8), 7.06-7.43 (21H, m, 3×*Ph*CH₂O-; *Ph*C=C- and H-3).
δ_{C} (125 MHz) : -4. 9, -4.5, 8.5, 18.2, 25.8, 45.6, 60.1 (C-5), 63.7 (C-8), 70.85 C-5*), 71.82, 73.22, 73.3 (C-6), 74.7, 81.1 (C-7), 83.3 (C≡C), 89.2 (C=C), 123.2 (C-3), 123.4, 124, 127.7, 127.8, 127.9, 128, 128.3, 128.5, 128.6,129, 130, 131.5, 137.5, 137.8, 137.9, 145.4 (C-8a).

**Compound 15:** A stock 1M solution of TBAF in THF (1 ml, 1 mmol) was added to a solution of **14** (0.289 g, 0.42 mmol) in THF (10 ml) at RT. The reaction was kept for 1 h at RT. The reaction was concentrated in vacuum. The residue was dissolved in DCM and washed with water. The aqueous layer was extracted with DCM once more. The combined organic layer was dried and evaporated. The brown residue was purified by flash column chromatography in Tol-EA 20→25% to give 0.183 g (0.32 mmol, 76 %) of the target product as yellow amorphous compound.
HRMS: Positive mode, m/z = 571.2590; expected for C₃₇H₃₅N₂O₄ [M+H]⁺ = 571.2597.
δ_{H} (500 MHz): 3.62 (2H, m, H-5*a, H-5*b), 3.88 (1H, dd, *J* = 3.2 Hz, *J* = 6.8 Hz, H-7), 4.05 (2H, m, H-5, H-6), 4.25 and 4.34 (2H, AB spectrum, *J*_{gem} = 12 Hz, PhC*H*₂O-), 4.41 and 4.72 (2H, AB spectrum, *J*_{gem} = 11.4 Hz, PhC*H*₂O-), 4.61 and 4.77 (2H, AB spectrum, *J*_{gem} = 12 Hz, PhC*H*₂O-), 5.1 (1H, d, *J* = 1.8 Hz, H-8), 7.0-7.3 (19H, m), 7.38 (2H, m).
δ_{C} (125 MHz): 58.5 (C-5), 61.8 (C-8), 70 (C-5*), 70.6, 71.6, 72.1, 72.5 (C-6), 72.9, 77.2 (c-7), 82.1 (C≡C), 88.4 (C=C), 121.6 (C-3), 122.26, 122.8, 124.3, 126.7, 126.8, 126.9, 127.2, 127.4, 127.7, 128, 130.4, 130.7, 136.3, 136.4, 136.7, 144.5 (C-8a).

**Compound 16:** To a solution of **15** (0.32 g, 0.56 mmol) in toluene (5ml) diphenylphosphoryl azide (0.614 ml, 2.85 mmol) was added followed by diazobicycloundecene (0.426 ml, 2.85 mmol) at RT. The reaction was kept for 4 h at RT. The reaction was diluted with THF to dissolve the oily sediment and heated-up to boil for 1min. The reaction was then concentrated in vacuum. The brown residue was purified by flash column chromatography in PE-EA 5→25% to give 0.317 g (0.53 mmol, 92 %) of the target product.
[α]_{D} = +31.8° c 1.0 CHCl₃
HRMS: Positive mode, m/z = 596.2627; expected for C₃₇H₃₄N₅O₃ [M+H]⁺ = 596.2662.
δ_{H} (500 MHz): 3.58 (1H, dd, *J*_{5a*,5} = 5.2 Hz, *J*_{5a*,5b*} = 10.4 Hz, H-5*a), 3.66 (1H, dd, *J*_{5b*,5} = 3 Hz, H-5*b), 3.81 (1H, dd, *J*_{7,8} = 7.1 Hz, *J*_{7,6} = 8.2 Hz, H-7), 3.86 (1H, dd, *J*_{6,5} = 7.5 Hz, H-6), 4.05 (1H, ddd, H-5), 4.3 and 4.35 (2H, AB spectrum, *J*_{gem} = 12 Hz, PhC*H*₂O-), 4.44 and 4.78 (2H, AB spectrum, *J*_{gem} = 11.2 Hz, PhC*H*₂O-), 4.61 (1H, d, H-8), 4.71 and 4.78 (2H, AB spectrum, *J*_{gem} = 11Hz, PhC*H*₂O-), 7.05-7.5 (21H, m, 3×*Ph*CH₂O-; *Ph*C=C- and H-3).
δ_{C} (75 MHz): 58.9 (C-8), 59.1 (C-5), 68.5 (C-5*), 73.35, 74.6, 74.9, 75.1 (C-6), 80.6 (C-7), 82.9(C≡C), 89.6 (C≡C), 122 (C-3), 123.3, 125.24, 128, 128.1, 128. 16, 128.2, 128.3, 128.6, 131.55, 137.1, 137.2, 141.4 (C-8a).

**Compound 17**: To a solution of **16** (0.12 g, 0.201 mmol) in THF-H₂O 10:1 (2 ml) triphenylphosphine (0.11 g, 0.42 mmol) was added at RT. The reaction was kept for 3 h at +60°C. The reaction was cooled down to RT and isobutyric anhydride (0.1 ml, 0.6 mmol) was added followed by triethylamine (0.2 ml, 1.4 mmol). The resulting turbid solution was kept RT for 1.5 h and then evaporated with toluene (2×10ml). The residue was purified by flash column chromatography in PE-EA 5→25% to give 0.12 g (0.19 mmol, 95 %) of the target product.
HRMS: Positive mode, m/z = 640.3168; expected for C₄₁H₄₂N₃O₄ [M+H]⁺ = 640.3175.
δ_{H} (500 MHz): 0.99 and 1.03 (6H, 2xd, *J* = 7.8 Hz, (*CH*₃)₂CH-), 2.2 (1H, quint, (*CH*₃)₂C*H*-), 3.67 (2H, m, H-5*a, H-5*b), 3.93 (1H, dd, *J*_{6,5} = 2.6 Hz, *J*_{6,7} = 4.7 Hz, H-6), 4.05 (1H, dd, *J*_{7,8} = 2.3 Hz, H-7), 4.34 and 4.42 (2H, AB spectrum, *J*_{gem} = 11.7 Hz, PhC*H*₂O-), 4.4 (1H, m, H-5), 4.47 (2H, AB spectrum, PhC*H*₂O-), 4.62 and 4.83 (2H, AB spectrum, *J*_{gem} = 11.7 Hz, PhC*H*₂O-), 5.31 (1H, dd, *J*_{8,NH} = 7.6 Hz , H-8), 6.18 (1H, bd, (C*H*₃)₂CHCON*H*-), 7.09 (2H, m), 7.15-7.31 (17H, m), 7.44 (2H, m).
δ_{C} (125 MHz): 19.1 and 19.6 (*C*H₃)₂CH-), 35.3 (CH₃)₂*C*H-), 46.2 (C-8), 59 (C-5), 71.3 (C-5*), 72.5, 72.7, 73.5, 74.3 (C-6), 76.1 (C-7), 82.9 (C≡C), 89.6 (C≡C), 123.3 (C-2), 124.2, 127.7, 127.8, 127.9, 128, 128.3, 128.4, 128.6, 131.5, 136.8, 137.4, 137.6, 141.8 (C-8a), 176.1 (CH₃)₂CHCONH-).

**Compound II:** A solution of **17** (0.1 g, 0.16 mmol) in acetic acid (3 ml) was added to a slurry of Pd(OH)₂/C (0.5 g) in acetic acid (2ml) preliminary activated by stirring under slight H₂ overpressure for 30 min at RT. The reaction flask was flushed with H₂ and the reaction was stirred under slight H₂ overpressure for 5h at RT. The reaction was filtered through a pad of Celite with the aid of MeOH and the filtrate was evaporated to dryness. The residue was purified by flash column chromatography in gradient CHCl₃-MeOH 5→20% to give 0.036 g (0.096 mmol, 60 %) of the target product.
HRMS: Positive mode, m/z = 374.2075; expected for C₂₀H₂₈N₃O₄ [M+H]⁺ = 374.2080.
δ_{H} (500 MHz, Pyridine d₅): 1.18 and 1.19 (6H, 2×d, *J* = 6.8 Hz, (C*H*₃)₂CH-), 2.66 (1H, quint, (CH₃)₂C*H*-), 2.95 (4H, m,-C*H*₂C*H*₂-), 4.2 (1H, m, H-5), 4.3 (2H, m, H-5*a, H-7), 4.47 (1H, dd, *J*_{6,5} = 8.6 Hz, *J*_{6,7} = 8.6 Hz, H-6), 4.55 (1H, dd, *J*_{5*a,5*b} = 11.7 Hz, *J*_{5*b,6} = 2.6 Hz, H-5*b), 5.73 (1H, dd, *J*_{8,7} = 8.4 Hz, *J*_{8,NH} = 8.4 Hz, H-8), 7.1 (1H, m), 7.17-7.2 (4H, 2×s), 7.28 (1H, bs, H-3), 8.81 (1H, bd, (C*H*₃)₂CHCON*H*-),
δ_{C} (125 MHz): 21.9 and 22.1 ((CH₃)₂CH-), 33.3 (-*C*H₂-), 37.7 (C*H*₃)₂*C*H-), 38.3 (-*C*H₂-), 53.7 (C-8), 64.1 (C-5*), 64.8 (C-5), 72.3 (C-6), 77.6 (C-7), 116.5 (C-3), 128.1, 130.7, 130.8, 144.4, 144.9, 146.4, 180.1.
Compounds III and IV were prepared by the same synthetic route, with appropriate changes to the acylating agent used as discussed above. The compounds were found to have the following physical characteristics.

**Compound III:** HRMS: Positive mode, m/z = 346.1761; expected for C₁₈H₂₄N₃O₄ [M+H]⁺ = 346.1767.
δ_{H} (500 MHz, CDCl₃-CD₃OD): 1.86 (3H, s, C*H₃*CO-), 2.57 (2H, m, (-CH₂C*H*₂-), 2.66 (2H, m, -C*H*₂CH₂-), 3.53 (1H, dd, *J*_{6,7} = 8.5 Hz, *J*_{7,8} = 8.3 Hz, H-7), 3.63 (2H, m, H-5, H-6), 3.71 (1H, dd, *J*_{5*a,5*b} = 12.2 Hz, *J*_{5*a,6} = 4 Hz, H-5*a), 3.86 (1H, dd, *J*_{5*b,6} = 2.8 Hz, H-5*b), 4.68 (1H, d, H-8), 6.65 (1H, s, H-3), 6.95 (3H, m), 7.03 (2H, m).

**Compound IV:** HRMS: Positive mode, m/z = 360.1922; expected for C₁₉H₂₆N₃O₄ [M+H]⁺ = 360.1923.
δ_{H} (500 MHz, CDCl₃-CD₃OD): 1.1 (3H, t, C*H₃*CH*₂*CO-), 2.44 (2H, m, C*H₃*CH*₂*CO-), 2.71 (2H, m, (-CH₂C*H₂*-), 2.78 (2H, m, -C*H*₂CH₂-), 3.7 (1H, dd, *J*_{6,7} = 8.5 Hz, *J*_{7,8} = 8.1 Hz, H-7), 3.78 (2H, m, H-5, H-6), 3.85 (1H, bd, *J*_{5*a,5*b} = 12.2 Hz, H-5*a), 4.04 (1H, H-5*b), 4.85 (1H, d, H-8), 6.87 (1H, s, H-3), 7.08 (3H, m), 7.15 (2H, m).

### Example 2

Compound II of the invention was used in kinetic studies against a bacterial *O*-GlcNAcase (OGA) (NagJ from *Clostridium perfringens;* Reference 12) and against a mixture of the human placental hexosaminidases HexA/HexB.

The results, as determined by the methods described below show (Reference 12) that compound II is 100,000 fold selective for the *O*-GlcNAcase active site in comparison to the HexA/HexB enzymes.

### Enzymology

Steady state kinetics of bOGA were determined using the fluorogenic substrate 4-methylumbelliferyl- *N*-acetyl-β-D-glucosaminide (4MU-NAG; Sigma). Standard reaction mixtures (50 µl) contained 2 pM bOGA in 50 mM citric acid, 125 mM NaHPO₄, 0.1 mg/ml BSA, and 1.5 - 25 µM of substrate in water. The reaction mixture was incubated for 466 min at 20 °C (RT). The reaction was stopped by the addition of a 2-fold excess (100 µl) of 3 M glycine-NaOH, pH 10.3. The fluorescence of the released 4-methylumbelliferone was quantified using using a FLX 800 Microplate Fluorescence Reader (Bio-Tek), with excitation and emission wavelengths of 360 and 460 nm, respectively. The production of 4-methylumbelliferone was linear with time for the incubation period used, and less than 10% of the available substrate was hydrolysed. Experiments were performed in triplicate; all of the spectra were corrected for the background emission from the buffer and the protein. Michaelis-Menten parameters were obtained by fitting the fluorescence intensity with GraFit [ref 19].

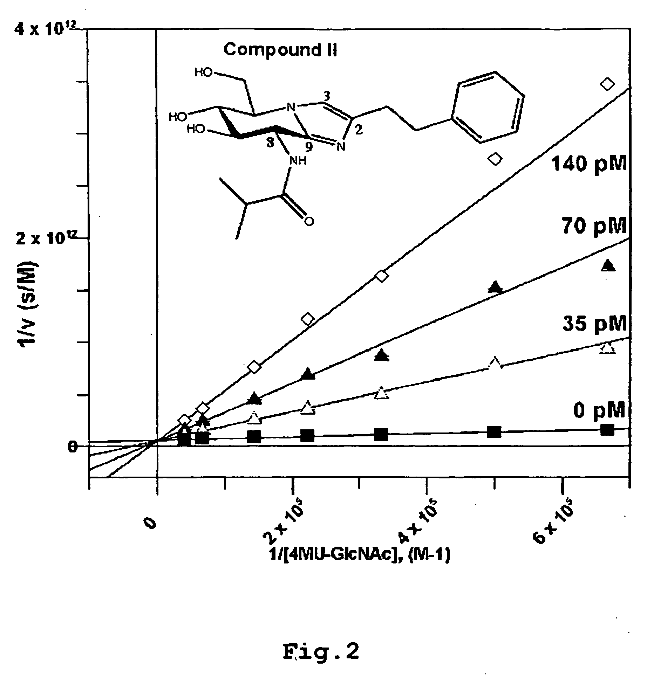

Determination of compound II Kᵢ was performed by steady-state kinetics in the presence of different concentrations (0, 35, 70, 140 pM) of the inhibitor. The mode of inhibition was visually verified by the Lineweaver-Burk plot (Figure 2), and the Kᵢ determined by fitting all fluorescence intensity data to the standard equation for competitive inhibition in GraFit [Ref 19]. Using this approach, the following parameters were determined from the data shown: *K*ᵢ = 4.6 ± 0.2 pM, *K*ₘ = 2.6 ± 0.1 µM, *K*_{cat} = 8.1 ± 0.5 s⁻¹.

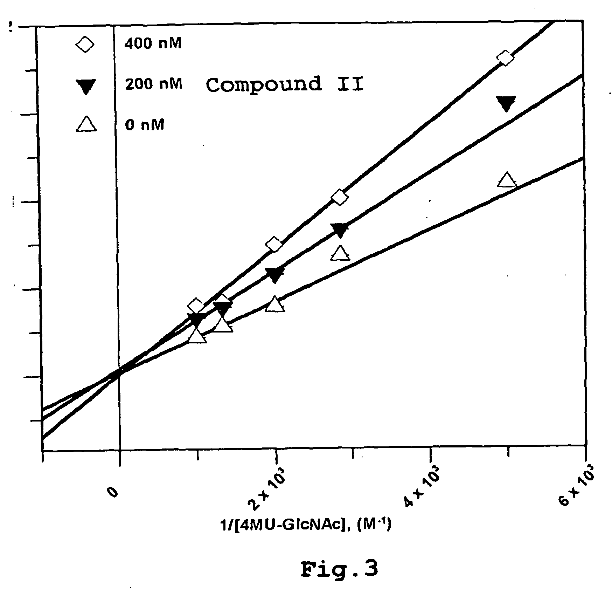

Steady state kinetics of hexosaminidases A and B (purchased from Sigma, catalogue number A6152), were determined using the fluorogenic substrate 4MU-GlcNAc substrate described above, using the same standard reaction mixture, with the following changes: 5*10⁻⁶ units / ml enzyme mixture was used with a varying substrate concentration between 200 and 1000 µM. Determination of the compound II Kᵢ for placental hexosaminidases was performed by steady-state kinetics in the presence of different concentrations (0, 200, 400 nM) of the inhibitor, and interpreted as for bOGA, with the data shown in Figure 3. The following parameters were determined from the data shown: *K*ᵢ = 0.52 ± 0.07 µM, *K*ₘ = 400 ± 40 µM.

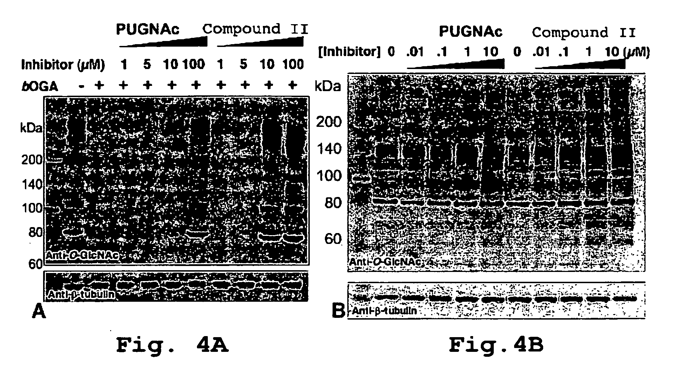

To evaluate the usefulness of compound II as a chemical tool to study the effects of inhibition of OGA in human cells, activity of the compound in human SH-SY5Y human neuroblastoma cell lysates and the HEK 293 cell line was evaluated. Fig. 4A shows that compound II is more active than PUGNAc in inhibiting bOGA from removing *O*-GlcNAc from proteins in SH-SY5Y cell lysates. More importantly, when used to treat HEK 293 cells, compound II was more efficient at raising *O*-GlcNAc levels than PUGNAc (Fig. 4B).

### Example 3

The Kᵢ against both a bacterial *O*-GlcNAcase (bOGA) (NagJ from *Clostridium perfringens;* Reference 12) and against a mixture of the human placental hexosaminidases HexA/HexB was obtained for each of Compounds III, IV and V in the same manner as described above in example 2 for compound II. The results are shown in the Table below together with those for compound II.

**Table 1**

| **Ki values (in M)** | **Compound V** | **Compound III** | **Compound II** | **Compound IV** |
|---|---|---|---|---|
| **a) hexosaminidases** | **7.5×10⁻⁹** | **2.0×10⁻⁹** | **0.5×10⁻⁶** | **4.8×10⁻⁹** |
| **b) bOGA** | **3.0×10⁻¹²** | **7.8×10⁻¹²** | **4.6×10⁻¹²** | **11.0×10⁻¹²** |
| **Ratio a/b** | **2500** | **256** | **108000** | **440** |

These results illustrate the selectivity of compounds general formula I for the *O*-GlcNAcase active site in comparison to the HexA/HexB enzymes. Compound II exhibits a particularly high selectivity with the ratio between the Ki values found in excess of 100,000.

### Example 4

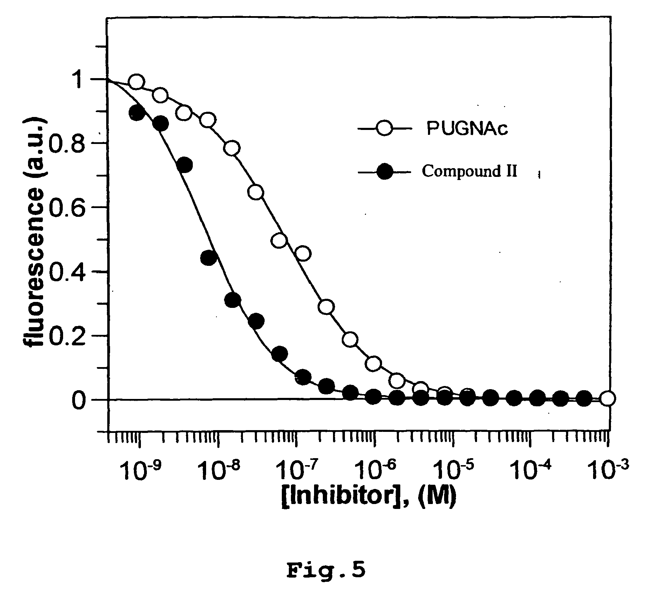

A comparison of the inhibition of human OGA (hOGA) by Compound II and PUGNAc is shown in Figure 5. The methodology used was similar to that described above for bOGA. The graph illustrates the effect of increasing concentration of each of the inhibitors on the enzyme activity of OGA measured in fluorescence absorbance units and demonstrates that GlcNacstain is a more potent inhibitor than PUGNac. The IC50 results obtained were: PUGNAc: IC50: 71 nM +- 6 nM
GlcNAcstatin C: 8 nM +- 0.7 nM.

### References

(1) Torres, C. R.; Hart, G. W. J.Biol.Chem. 1984, 259, 3308-3317.
(2) Zachara, N. E.; Hart, G. W. Biochim.Biophys.Acta 2004, 1673, 13-28.
(3) Love, D. C.; Hanover, J. A. Science STKE 2005, 312, 1-14.
(4)Wells, L.; Gao, Y.; Mahoney, J. A.; Vosseller, K.; C hen, C.; Rosen, A.; Hart, G. W. J.Biol.Chem. 2002, 277, 1755-1761.
(5)Lehman, D. M.; Fu, D. J.; Freeman, A. B.; Hunt,K. J.; Leach, R. J.; Johnson-Pais, T.; Hamlington, J.; Dyer, T. D.; Arya, R.; Abboud, H.; Goring, H. H.; Duggirala, R.; Blangero, J.; Konrad, R. J.; Stern, M. P. Diabetes 2005, 54, 1214-1221.
(6) http://afmb.cnrs-mrs.fr/CAZY
(7)Horsch, M.; Hoesch, L.; Vasella, A.; Rast, D. M. Eur.J.Biochem. 1991, 197, 815-818.
(8)Haltiwanger, R. S.; Grove, K.; Philipsberg, G. A. J.Biol.Chem. 1998, 273, 3611-3617.
(9)Macauley, M. S.; Whitworth, G. E.; Debowski, A. W.; C hin, D.; Vocadlo, D. J. J.Biol.Chem. 2005, 280, 25313-25322.
(10)Stubbs, K. A.; Zhang, N.; Vocadlo, D. J. Org.Biomol.Chem. 2006, 4, 839-845.
(11a) Shanmugasundaram, B.; Debowski, A.W.; Dennis, R.J.; Davies, G.J.; Vocadlo D.J.; Vasella, A. Chem.Commun. 2006, 4372-4374.
(11)Kim, E. J.; Perreira, M.; Thomas, C. J.; Hanover, J. A. J.Am.Chem.Soc. 2006, 128, 4234-4235.
(12) Rao, F. V.; Dorfmueller, H. C.; Villa, F.; Allwood, M.; Eggleston, I. M.; van Aalten, D. M. F. EMBO J. 2006, 25, 1569-1578.
(13) Terinek, M.; Vasella, A. Helv.Chim. Acta 2005, 88, 10-22.
(14)Aoyagi, T.; Suda, H.; Uotani, K.; Kojima, F.; Aoyam a, T.; Horiguchi, K.; Hamada, M.; Takeuchi, T. J.Antibiot. 1992, 45, 1404-1408.
(15) Tatsuta, K.; Miura, S. Tetrahedron Lett. 1995, 36, 6721-6724.
(16)Tatsuta, K.; Miura, S.; Ohta, S.; Gunji, H. J.Antibiot. 1995, 48, 286-288.
(17)Tatsuta, K.; Miura, S.; Ohta, S.; Gunji, H. Tetrahedron Lett. 1995, 36, 1085-1088.
(18)Heightman, T. D.; Vasella, A. T. Angew.Chem.-Int.Edit. 1999, 38, 750-770.
(19) Leatherbarrow, R. J. GraFit Version 5, Erithacus Software Ltd. , Horley, U.K. 2001**, .**
(20) Terinek, M.; Vasella,A. Helvetica Chimica Acta 2005, 10-22.
(21) Desvergnes, S.; Py, S.; Vallee, Y. J. Org. Chem. 2005, 70, 1459-1462.
(22) Secrist, J. A., III; Tiwari, K. N.; Shortnacy-Fowler, A. T.; Messini, L.; Riordan, J. M.; Montgomery, J. A.; Meyers, S. C.; Ealick, S. E. J. Med. Chem. 1998, 41, 3865-3871.

## Claims

1. A compound of general formula I or a pharmaceutically acceptable salt thereof: 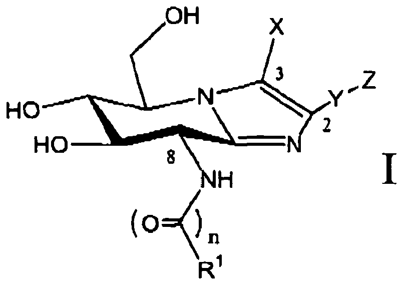 wherein n is 0 or 1;
X is hydrogen, halogen, -OH, -SH, -NH₂; and
R¹ is a C₁ - C₆ saturated or unsaturated aliphatic chain which may be branched, a five membered, saturated or unsaturated ring, comprising from one to four nitrogen atoms or a substituted derivative thereof, 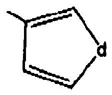 where d is CH₂, O or S; or, when n is 1, 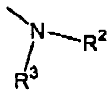 where R², R³ are independently hydrogen or C₁ - C₅ alkyl or are fused to form a saturated ring with the nitrogen which has from two to five carbon atoms;
-Y- is a linking group selected from the group consisting of -CH₂CH₂-, -CH=CH-, -C≡C-, -CH₂O-, -CH₂S- and -CH₂NH-; and
-Z is a hydrophobic group selected from: aryl, substituted aryl, heteroaryl, substituted heteroaryl, C₁-C₆ saturated or unsaturated alkyl which may be branched, C₅ - C₆ saturated or unsaturated cycloalkyl or -COOR⁴ where R⁴ is C₁ - C₆ saturated or unsaturated alkyl which may be branched, aryl, or heteroaryl; wherein the term substituted refers to substitution with one or more of alkyl, halogen, hydroxyl, carboxyl, carboxylate ester, cyano, amino, nitro, oxo, or thio, the term aryl refers to C₅-C₁₄ aromatic radicals, and the term heteroaryl refers to C₆-C₁₄ aromatic radicals comprising at least one atom that is not carbon;
for use in the treatment or prophylaxis of Alzheimers Disease, diabetes or cancer.

2. The compound or pharmaceutically acceptable salt for the use according to claim 1 wherein the linking group -Y-is -CH₂CH₂-.

3. The compound or pharmaceutically acceptable salt for the use according to claim 1 or claim 2 wherein the group Z is phenyl or substituted phenyl.

4. The compound or pharmaceutically acceptable salt for the use according to any one of claims 1 to 3 wherein R¹ is a five membered ring of the form 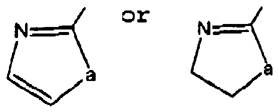 where a is O, S or NH.

5. The compound or pharmaceutically acceptable salt for the use according to any one of claims 1 to 3 wherein when n is 1 and R¹ has the form, 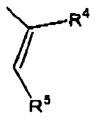 wherein R₄ and R5 are, independently, hydrogen or methyl.

6. The compound or pharmaceutically acceptable salt for the use according to claim 1 wherein the compound of general formula I is a compound of formula II:

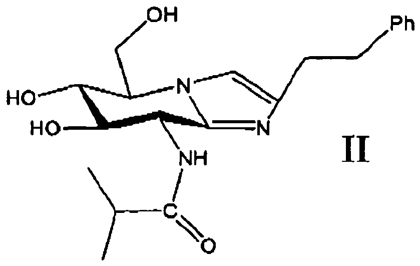

7. The compound or pharmaceutically acceptable salt for the use according to claim 1 wherein the compound of general formula I is a compound of formula III or of formula IV:

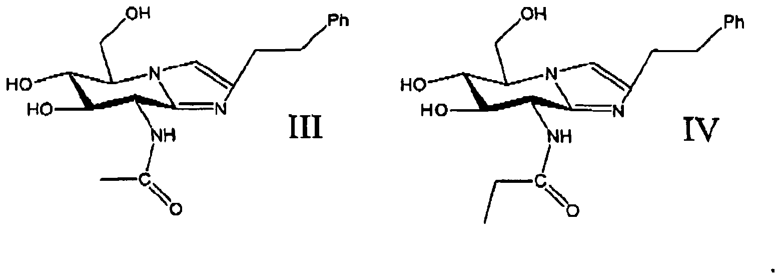

8. Use of a compound or pharmaceutically acceptable salt thereof as defined in any one of claims 1 to 7 in the preparation of a medicament for treatment or prophylaxis of Alzheimers Disease, diabetes or cancer.

9. An in vitro method of inhibiting *O*-GlcNAcase enzymes comprising the step of administering a compound of general formula I: 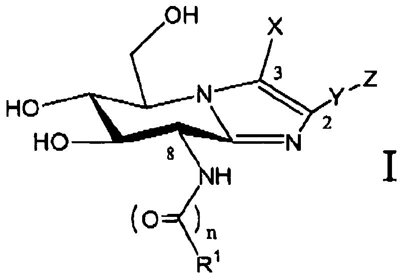 wherein n is 0 or 1;
X is hydrogen, halogen, -OH, -SH, -NH₂; and
R¹ is a C₁ - C₆ saturated or unsaturated aliphatic chain which may be branched, a five membered, saturated or unsaturated ring, comprising from one to four nitrogen atoms or a substituted derivative thereof, 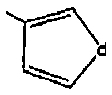 where d is CH₂, O or S; or, when n is 1, 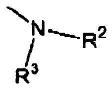 where R², R³ are independently hydrogen or C₁ - C₅ alkyl or are fused to form a saturated ring with the nitrogen which has from two to five carbon atoms;
-Y- is a linking group selected from the group consisting of -CH₂CH₂-, -CH=CH-, -C≡C-, -CH₂O-, -CH₂S- and CH₂NH-; I and
-Z is a hydrophobic group selected from: aryl, substituted aryl, heteroaryl, substituted heteroaryl, C₁ - C₆ saturated or unsaturated alkyl which may be branched, C₅ - C₆ saturated or unsaturated cycloalkyl or-COOR⁴ where R⁴ is C₁ - C₆ saturated or unsaturated alkyl which may be branched, aryl, or heteroaryl; wherein the term substituted refers to substitution with one or more of alkyl, halogen, hydroxyl, carboxyl, carboxylate ester, cyano, amino, nitro, oxo, or thio, the term aryl refers to C₆-C₁₄ aromatic radicals, and the term heteroaryl refers to C₆-C₁₄ aromatic radicals comprising at least one atom that is not carbon;
to a cell or an extract containing O-GlcNAcase enzymes.

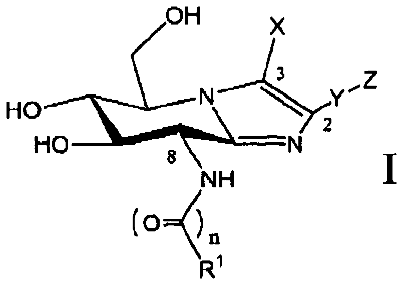

10. A compound of formula II or formula IV: 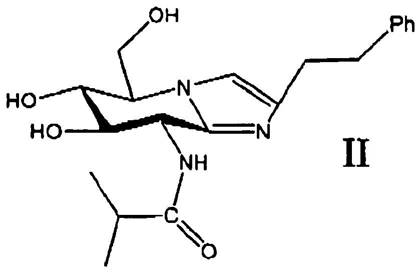 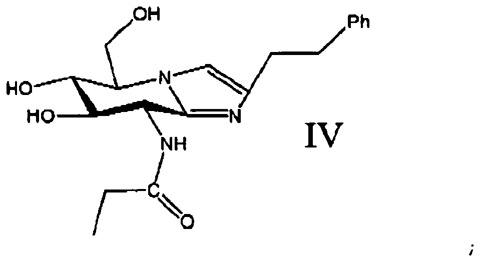 or a pharmaceutically acceptable salt thereof.

11. A pharmaceutical composition comprising a compound of formula I according to claim 10, or a pharmaceutically acceptable salt thereof; together with a pharmaceutically acceptable carrier therefor.

12. A compound according to claim 10, or a pharmaceutically acceptable salt thereof; for use in therapy.

13. A method for the synthesis of compounds according to general formula VI: 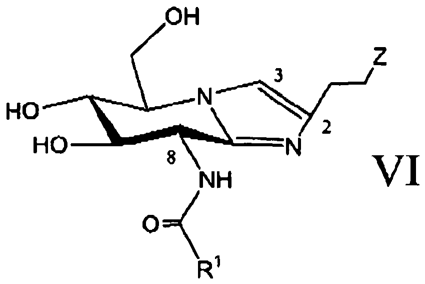 where Z and R¹ have the same meaning as in claim 1, comprising the steps of :
a) reacting a compound of formula VII: 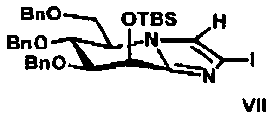 with a compound of formula VIII: 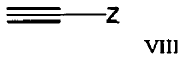 in the presence of a palladium catalyst such as Pd(PPh₃)₄ and then removing the TBS protecting group with TBAF tetrabutylammonium fluoride to afford a compound of formula IX: 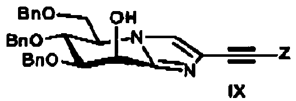
b) reacting the compound of formula IX with DPPA (diphenylphosphorylazide),DHU (1,8-diazabicyclo [5.4.0] undec-7-ene) to form the azido compound X; 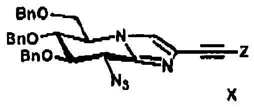
c) reducing the azide with PPh₃ to the amine and acylating with a compound of formula XI (R₁CO)₂O, to give a compound of formula XII: 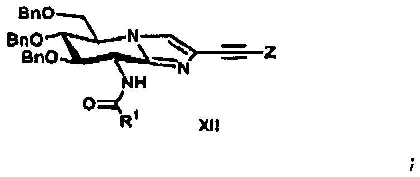 and
d) saturating the triple bond and removing the benzyl protecting groups to afford the compounds of formula VI.

## Patentansprüche

1. Verbindung der allgemeinen Formel I oder pharmazeutisch verträgliches Salz davon 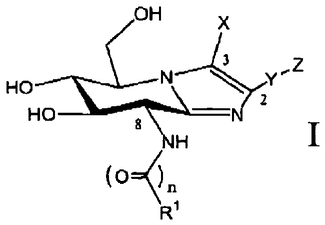 wobei n 0 oder 1 beträgt;
X Wasserstoff, Halogen, -OH, -SH, -NH₂ ist; und
R¹ eine gesättigte oder ungesättigte aliphatische C₁-C₆-Kette, die verzweigt sein kann, ein fünfgliedriger, gesättigter oder ungesättigter Ring, der ein bis vier Stickstoffatome umfasst, oder ein substituiertes Derivat davon ist, 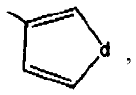 wobei d CH₂, O oder S ist; oder, wenn n 1 beträgt, 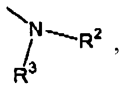 wobei R², R³ unabhängig Wasserstoff oder C₁-C₅-Alkyl sind oder kondensiert sind, um einen gesättigten Ring mit dem Stickstoff zu bilden, der zwei bis fünf Kohlenstoffatome aufweist;
-Y- eine Verknüpfungsgruppe ist ausgewählt aus der Gruppe bestehend aus-CH₂CH₂-, -CH=CH-, -C≡C-, -CH₂O-, -CH₂S- und -CH₂NH-; und
-Z eine hydrophobe Gruppe ist ausgewählt aus: Aryl, substituiertem Aryl, Heteroaryl, substituiertem Heteroaryl, gesättigtem oder ungesättigtem C₁-C₆-Alkyl, das verzweigt sein kann, gesättigtem oder ungesättigtem C₅-C₆-Cycloalkyl oder -COOR⁴, wobei R₄ gesättigtes oder ungesättigtes C₁-C₆-Alkyl, das verzweigt sein kann, Aryl oder Heteroaryl ist; wobei der Ausdruck substituiert sich auf eine Substitution mit einem oder mehreren von Alkyl, Halogen, Hydroxyl, Carboxyl, Carboxylatester, Cyano, Amino, Nitro, Oxo oder Thio bezieht, der Ausdruck Aryl sich auf aromatische C₆-C₁₄-Radikale bezieht und der Ausdruck Heteroaryl sich auf aromatische C₆-C₁₄-Radikale, die mindestens ein Atom, das nicht Kohlenstoff ist, umfassen, bezieht;
zur Verwendung bei der Behandlung oder Prophylaxe von Alzheimer-Krankheit, Diabetes oder Krebs.

2. Verbindung oder pharmazeutisch verträgliches Salz zur Verwendung nach Anspruch 1, wobei die Verknüpfungsgruppe -Y- -CH₂CH₂- ist.

3. Verbindung oder pharmazeutisch verträgliches Salz zur Verwendung nach Anspruch 1 oder Anspruch 2, wobei die Gruppe Z Phenyl oder substituiertes Phenyl ist.

4. Verbindung oder pharmazeutisch verträgliches Salz zur Verwendung nach einem der Ansprüche 1 bis 3, wobei R¹ ein fünfgliedriger Ring der Form 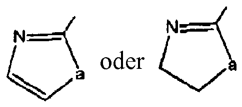 ist, wobei a O, S oder NH ist.

5. Verbindung oder pharmazeutisch verträgliches Salz zur Verwendung nach einem der Ansprüche 1 bis 3, wobei, wenn n 1 beträgt und R¹ die Form 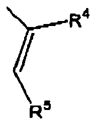 aufweist, wobei R4 und R5 unabhängig Wasserstoff oder Methyl sind.

6. Verbindung oder pharmazeutisch verträgliches Salz zur Verwendung nach Anspruch 1, wobei die Verbindung der allgemeinen Formel I eine Verbindung der Formel II: 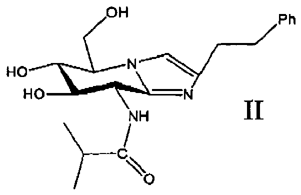 ist.

7. Verbindung oder pharmazeutisch verträgliches Salz zur Verwendung nach Anspruch 1, wobei die Verbindung der allgemeinen Formel I eine Verbindung der Formeln III oder der Formel IV: 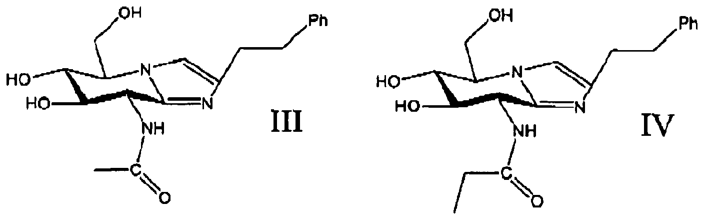 ist.

8. Verwendung einer Verbindung oder eines pharmazeutisch verträglichen Salzes davon wie in einem der Ansprüche 1 bis 7 definiert, bei der Herstellung eines Medikaments zur Behandlung oder Prophylaxe von Alzheimer-Krankheit, Diabetes oder Krebs.

9. In vitro-Verfahren zum Hemmen von O-GlcNAcase-Enzymen, umfassend den Schritt des Verabreichens einer Verbindung der allgemeinen Formel I: 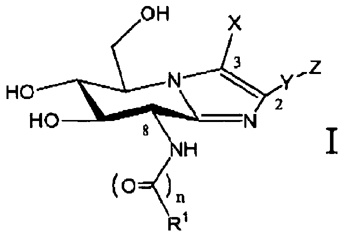 wobei n 0 oder 1 beträgt;
X Wasserstoff, Halogen, -OH, -SH, -NH₂ ist; und
R¹ eine gesättigte oder ungesättigte aliphatische C₁-C₆-Kette, die verzweigt sein kann, ein fünfgliedriger, gesättigter oder ungesättigter Ring, der ein bis vier Stickstoffatome umfasst, oder ein substituiertes Derivat davon ist, 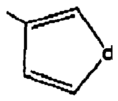 wobei d CH₂, O oder S ist; oder, wenn n 1 beträgt, 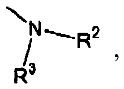 wobei R², R³ unabhängig Wasserstoff oder C₁-C₅-Alkyl sind oder kondensiert sind, um einen gesättigten Ring mit dem Stickstoff zu bilden, der zwei bis fünf Kohlenstoffatome aufweist;
-Y- eine Verknüpfungsgruppe ist ausgewählt aus der Gruppe bestehend aus-CH₂CH₂-, -CH=CH-, -C≡C-, -CH₂O-, -CH₂S- und -CH₂NH-; und
-Z eine hydrophobe Gruppe ist ausgewählt aus: Aryl, substituiertem Aryl, Heteroaryl, substituiertem Heteroaryl, gesättigtem oder ungesättigtem C₁-C₆-Alkyl, das verzweigt sein kann, gesättigtem oder ungesättigtem C₅-C₆-Cycloalkyl oder -COOR⁴, wobei R⁴ gesättigtes oder ungesättigtes C₁-C₆-Alkyl, das verzweigt sein kann, Aryl oder Heteroaryl ist; wobei der Ausdruck substituiert sich auf eine Substitution mit einem oder mehreren von Alkyl, Halogen, Hydroxyl, Carboxyl, Carboxylatester, Cyano, Amino, Nitro, Oxo oder Thio bezieht, der Ausdruck Aryl sich auf aromatische C₆-C₁₄-Radikale bezieht und der Ausdruck Heteroaryl sich auf aromatische C₆-C₁₄-Radikale, die mindestens ein Atom, das nicht Kohlenstoff ist, umfassen, bezieht;
an eine Zelle oder einen Extrakt, die/der O-GlcNAcase-Enzyme enthält.

10. Verbindung der Formel II oder Formel IV: 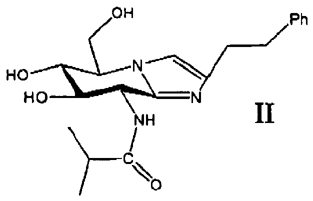 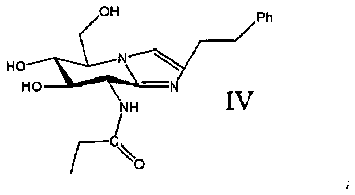 oder pharmazeutisch verträgliches Salz davon.

11. Pharmazeutische Zusammensetzung umfassend eine Verbindung der Formel I nach Anspruch 10 oder pharmazeutisch verträgliches Salz davon; zusammen mit einem pharmazeutisch verträglichen Träger dafür.

12. Verbindung nach Anspruch 10 oder pharmazeutisch verträgliches Salz davon; zur Verwendung in der Therapie.

13. Verfahren zur Synthese von Verbindungen nach der allgemeinen Formel VI: 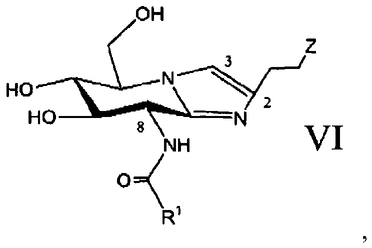 wobei Z und R¹ dieselbe Bedeutung wie in Anspruch 1 aufweisen, umfassend die Schritte des:
a) Reagierens einer Verbindung der Formel VII: 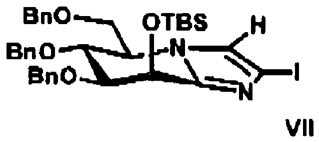 mit einer Verbindung der Formel VIII: 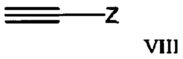 in Gegenwart eines Palladium-Katalysators, wie beispielsweise Pd(PPh₃)₄, und dann Entfernens der TBS-Schutzgruppe mit TBAF Tetrabutylammoniumfluorid, um eine Verbindung der Formel IX: 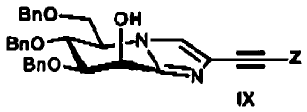 zu liefern,
b) Reagierens der Verbindung der Formel IX mit DPPA (Diphenylphosphorylazid), DBU (1,8-Diazabicyclo[5.4.0]undec-7-en), um die Azidoverbindung X: 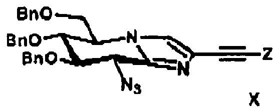 zu bilden,
c) Reduzierens des Azids mit PPh₃ zu dem Amin und Acylierens mit einer Verbindung der Formel XI (R₁CO)₂O, um eine Verbindung der Formel XII: 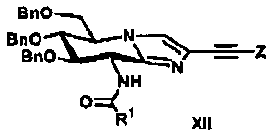 zu ergeben; und
d) Sättigens der Dreifachbindung und Entfernens der Benzylschutzgruppen, um die Verbindungen der Formel VI zu liefern.

## Revendications

1. Composé de formule générale I ou sel pharmaceutiquement acceptable de celui-ci: 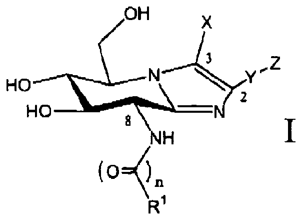 dans laquelle n a la valeur de 0 ou 1;
X est un atome d'hydrogène, un groupe halogéno, -OH, -SH, -NH₂; et
R¹ est une chaîne aliphatique saturée ou insaturée en C₁ à C₆ qui peut être ramifiée, un cycle saturé ou insaturé, à cinq chaînons, comprenant de un à quatre atome(s) d'azote ou un dérivé substitué de celui-ci, 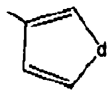 où d est CH₂, O ou S; ou, lorsque n a la valeur de 1, 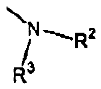 où R², R³ sont indépendamment un atome d'hydrogène ou un groupe alkyle en C₁ à C₅ ou sont condensés pour former un cycle saturé avec l'azote qui a de deux à cinq atomes de carbone;
-Y- est un groupe de liaison sélectionné parmi le groupe constitué de -CH₂CH₂-,-CH=CH-, -C≡C-, -CH₂O-, -CH₂S- et -CH₂NH-; et
-Z est un groupe hydrophobe sélectionné parmi: un groupe aryle, aryle substitué, hétéroaryle, hétéroaryle substitué, alkyle saturé ou insaturé en C₁ à C₆ qui peut être ramifié, cycloalkyle saturé ou insaturé en C₅ à C₆ ou -COOR⁴ où R⁴ est un groupe alkyle saturé ou insaturé en C₁ à C₆ qui peut être ramifié, aryle, ou hétéroaryle; dans lequel le terme substitué désigne une substitution par un ou plusieurs groupe(s) alkyle, halogéno, hydroxyle, carboxyle, ester carboxylate, cyano, amino, nitro, oxo, ou thio, le terme aryle désigne des radicaux aromatiques en C₆ à C₁₄, et le terme hétéroaryle désigne des radicaux aromatiques en C₆ à C₁₄ comprenant au moins un atome qui n'est pas un atome de carbone;
pour l'utilisation dans le traitement ou la prophylaxie de la maladie d'Alzheimer, du diabète ou du cancer.

2. Composé ou sel pharmaceutiquement acceptable pour l'utilisation selon la revendication 1 dans lequel le groupe de liaison -Y- est -CH₂CH₂-.

3. Composé ou sel pharmaccutiqucmcnt acceptable pour l'utilisation selon la revendication 1 ou la revendication 2 dans lequel le groupe Z est le groupe phényle ou phényle substitué.

4. Composé ou sel pharmaceutiquement acceptable pour l'utilisation selon l'une quelconque des revendications 1 à 3 dans lequel R¹ est un cycle à cinq chaînons de la forme 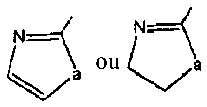 où a est O, S ou NH.

5. Composé ou sel pharmaceutiquement acceptable pour l'utilisation selon l'une quelconque des revendications 1 à 3 dans lequel lorsque n a la valeur de 1 et R¹ a la forme, 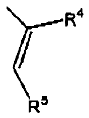 dans laquelle R⁴ et R⁵ sont, indépendamment, un atome d'hydrogène ou un groupe méthyle.

6. Composé ou sel pharmaceutiquement acceptable pour l'utilisation selon la revendication 1 dans lequel le composé de formule générale I est un composé de formule II:

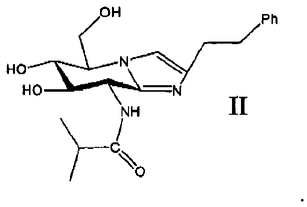

7. Composé ou sel pharmaceutiquement acceptable pour l'utilisation selon la revendication 1 dans lequel le composé de formule générale I est un composé de formule III ou de formule IV:

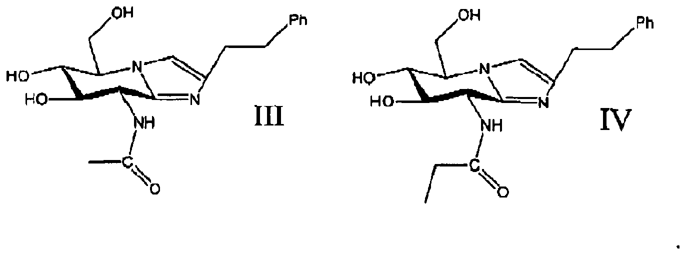

8. Utilisation d'un composé ou d'un sel pharmaceutiquement acceptable de celui-ci tel que défini selon l'une quelconque des revendications 1 à 7 dans la préparation d'un médicament pour le traitement ou la prophylaxic de la maladie d'Alzheimer, du diabète ou du cancer.

9. Procédé *in vitro* d'inhibition des enzymes O-GlcNAcase comprenant l'étape d'administration d'un composé de formule générale I: 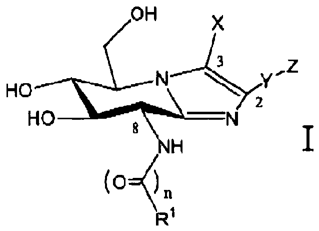 dans laquelle n a la valeur de 0 ou 1;
X est un atome d'hydrogène, un groupe halogéno, -OH, -SH, -NH₂; et
R¹ est une chaîne aliphatique saturée ou insaturée en C₁ à C₆ qui peut être ramifiée, un cycle saturé ou insaturé, à cinq chaînons, comprenant de un à quatre atome(s) d'azote ou un dérivé substitué de celui-ci, 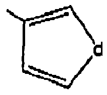 où d est CH₂, O ou S; ou, lorsque n a la valeur de 1, 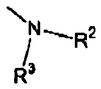 où R², R³ sont indépendamment un atome d'hydrogène ou un groupe alkyle en C₁ à C₅ ou sont condensés pour former un cycle saturé avec l'azote qui a de deux à cinq atomes de carbone;
-Y- est un groupe de liaison sélectionné parmi le groupe constitué de -CH₂CH₂-,-CH=CH-, -C≡C-, -CH₂O-, -CH₂S- et CH₂NH-; et
-Z est un groupe hydrophobe sélectionné parmi: un groupe aryle, aryle substitué, hétéroaryle, hétéroaryle substitué, alkyle saturé ou insaturé en C₁ à C₆ qui peut être ramifié, cycloalkyle saturé ou insaturé en C₅ à C₆ ou -COOR⁴ où R₄ est un groupe alkyle saturé ou insaturé en C₁ à C₆ qui peut être ramifié, aryle, ou hétéroaryle; dans lequel le terme substitué désigne une substitution par un ou plusieurs groupe(s) alkyle, halogéno, hydroxyle, carboxyle, ester carboxylate, cyano, amino, nitro, oxo, ou thio, le terme aryle désigne des radicaux aromatiques en C₆ à C₁₄, et le terme hétéroaryle désigne des radicaux aromatiques en C₆ à C₁₄ comprenant au moins un atome qui n'est pas un atome de carbone;
à une cellule ou un extrait contenant des enzymes O-GlcNAcases.

10. Composé de formule **II** ou de formule IV: 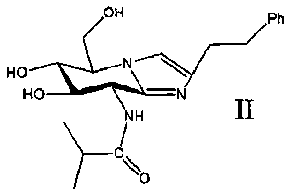 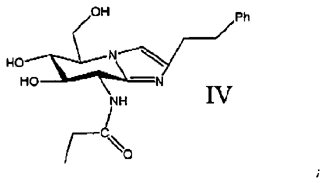 ou sel pharmaceutiquement acceptable de celui-ci.

11. Composition pharmaceutique comprenant un composé de formule I selon la revendication 10 ou sel pharmaceutiquement acceptable de celui-ci; conjointement à un support pharmaceutiquement acceptable pour celui-ci.

12. Composé selon la revendication 10 ou sel pharmaceutiquement acceptable de celui-ci; pour l'utilisation en thérapie.

13. Procédé de synthèse des composés selon la formule générale VI: 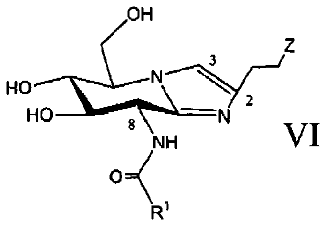 où Z et R¹ ont la même signification que dans la revendication 1, comprenant les étapes de:
a) réaction d'un composé de formule VII: 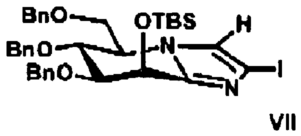 avec un composé de formule VIII: 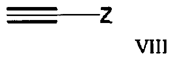 en la présence d'un
catalyseur au palladium tel que Pd(PPh₃)₄ et ensuite le retrait du groupe protecteur TBS avec du fluorure de tétrabutylammonium TBAF pour fournir un composé de formule IX: 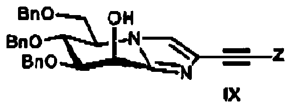
b) réaction du composé de formule IX avec du DPPA (azoture de diphénylphosphoryle), DBU (1,8-diazabicyclo[5.4.0]undéc-7-ène) pour former le composé azido X; 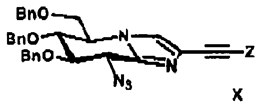
c) réduction de l'azoture avec PPh₃ en l'amine et acylation avec un composé de formule XI (R₁CO)₂O, pour donner un composé de formule XII: 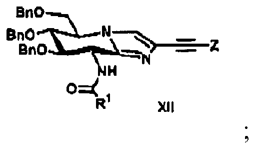 et
d) saturation de la triple liaison et retrait des groupes protecteurs benzyle pour fournir les composés de formule VI.
